# EUROPEAN PATENT SPECIFICATION

(11) **EP 1 983 903 B1**
(45) Date of publication and mention of the grant of the patent: **19.03.2014**
(21) Application number: 07717097.5
(22) Date of filing: 26.01.2007
(51) Int. Cl.: A61B 17/00, A61B 18/14

(54) **ABLATION DEVICE AND SYSTEM FOR GUIDING ABLATION DEVICE INTO A PATIENT'S BODY**
ABLATIONSVORRICHTUNG UND SYSTEM ZUM FÜHREN EINER ABLATIONSVORRICHTUNG IN DEN KÖRPER EINES PATIENTEN
DISPOSITIF D'ABLATION ET SYSTEME POUR GUIDER LE DISPOSITIF D'ABLATION DANS UN CORPS

(30) Priority: 27.01.2006 US 762699 P
(43) Date of publication of application: 29.10.2008
(73) Proprietor: Medtronic, Inc., Minneapolis, MN 55432-5604 (US)
(72) Inventor: KIM, David, Maple Grove, Minnesota 55311 (US); DAIGLE, Thomas, Corcoran, Minnesota 55374 (US); DICKERSON, Darrin, Blaine, Minnesota 55449 (US); SKARDA, James, Lake Elmo, Minnesota 55042 (US); PODBELSKI, Adam, St. Paul, Minnesota 55104 (US); BILITZ, Mark, Plymouth, Minnesota 55446 (US)
(74) Representative: Hughes, Andrea Michelle
(86) International application number: PCT/US2007/002332
(87) International publication number: WO 2007/089675

(56) References cited:
- WO-A-2005/120376
- US-A- 5 957 863
- US-A- 6 096 037
- US-A1- 2002 188 294
- US-A1- 2004 087 940
- US-A1- 2005 090 817
- US-A1- 2005 288 666

## Description

### FIELD OF THE INVENTION

The present description relates generally to the treatment of tissue of a patient with ablative energy and, more particularly, to an ablation device having a flexible shaft allowing for ease in surgical placement of the ablation device, and/or having a lockout feature that helps to prevent inadvertent application of ablative energy. The invention is set out in the appended claims.

### BACKGROUND OF THE INVENTION

Although the present description contemplates devices and systems relating to ablation of many types of tissue, in particular, the present application will focus on ablation devices and key features thereof, systems of guiding or placing ablation devices, and methods of using ablation devices and of guiding ablation devices into a body, for the ablation of heart tissue or tissue near the heart. Also, the present application contemplates the use of the described ablation devices, systems and methods to treat various conditions, however, the present application will focus particularly on treatment of heart arrhythmias (e.g., atrial fibrillation).

In a normal heart, contraction and relaxation of the heart muscle (myocardium) takes place in an organized fashion as electrochemical signals pass sequentially through the myocardium from the sinoatrial (SA) node located in the right atrium to the atrialventricular (AV) node and then along a well defined route which includes the His-Purkinje system into the left and right ventricles. Sometimes abnormal rhythms occur in the atrium which are referred to as atrial arrhythmia. Three of the most common arrhythmia are ectopic atrial tachycardia, atrial fibrillation, and atrial flutter. Arrhythmia can result in significant patient discomfort and even death because of a number of associated problems, including the following: (1) an irregular heart rate, which causes a patient discomfort and anxiety; (2) loss of synchronous atrioventricular contraction, which compromises cardiac hemodynamics resulting in varying levels of congestive heart failure; and (3) stasis of blood flow, which increases vulnerability to thromboembolism, It is sometimes difficult to isolate a specific pathological cause of the arrhythmia although it is believed that the principal mechanism is one or a multitude of stray circuits within the left and/or right atrium. These circuits or stray electrical signals are believed to interfere with the normal electrochemical signals passing from the SA node to the AV node and into the ventricles.

Treatment of arrhythmias may be accomplished by a variety of approaches, including drugs, surgery, implantable pacemakers/defibrillators, and catheter ablation. While arrhythmic drugs may be the treatment of choice for many patients, these drugs may only mask the symptoms and do not cure the underlying cause. Implantable devices, on the other hand, usually can correct an arrhythmia only after it occurs. Surgical and catheter-based treatments, by contrast, may actually cure the problem usually by ablating the abnormal arrhythmogenic tissue or abnormal pathway responsible for the arrhythmia. The catheter-based treatments rely on the application of various destructive energy sources to the target tissue including direct current energy sources to the target tissue including direct current electrical energy, radiofrequency electrical energy, microwave energy, laser energy, cryoenergy, ultrasound, and the like.

One surgical method of treating atrial fibrillation is the "Maze" procedure, which relies on a prescribed pattern of incisions to anatomically create a convoluted path, or maze, for electrical propagation within the left and right atria. The procedure employs incisions in the right and left atria which divide the atria into electrically isolated portions which in turn results in an orderly passage of a depolarization wave front from the SA node to the AV node, while preventing reentrant wave front propagation. The Maze procedure has been found very effective in curing arrhythmias. However, the procedure is technically difficult. The procedure also requires open heart surgery, in which the breastbone is divided and the surgeon has direct access to the heart.

More recently, Maze-like procedures have been developed utilizing ablation catheters that can form lesions on the endocardium to effectively create a maze for electrical conduction in a predetermined path. Typically, the lesions are formed by ablating tissue with an electrode carried by the catheter. Ablative energy, e.g., high intensity focused ultrasound (HIFU) energy, radiofrequency (RF) energy, microwave energy and/or laser energy, applied to the electrode, causes significant physiological effects in the tissue resulting from thermal and/or mechanical changes or effects. By controlling the energy level, the amount of heat generated in the tissue and the degree of tissue damage or change can also be controlled. Ablation uses lower levels of voltage that creates sufficient heat to cause a desired cell damage, but leaves the tissue structure intact so as to effectively block electrical pathways within the tissue. Irrigation of the electrode(s) during the ablation procedure with saline or other conductive fluid can decrease the interface impedance, cool the tissue, and allow for a greater lesion depth.

A treatment for atrial fibrillation, in particular, includes ablation around the pulmonary veins, which procedure is called pulmonary vein antrum isolation. Almost all the atrial fibrillation signals are believed to come from the four pulmonary veins and move to the atria. Ablation of the area of the atria that connects to the pulmonary veins provides circular scar tissue that blocks impulses firing within the pulmonary veins from moving to the atria, thereby disconnecting the pathway of abnormal rhythm and preventing atrial fibrillation.

Most previous ablation devices have been designed to access the heart via a midline sternotomy (i.e., an open surgical procedure). More recently, ablation of cardiac tissue can be carried out through a minimally invasive route, such as between the ribs, through a sub-xyphoid incision or via catheter that is introduced through a vein, and into the heart. Such minimally invasive procedures are generally performed off-pump, which means the heart is beating during the procedure. Such procedures generally require several ports for medical devices to enter the area of the heart and perform the procedures.

Ablation of a precise location within the heart requires precise placement of an ablation device within or near the heart. Precise positioning of the ablation device is especially difficult because of the physiology of the heart, particularly as such recently developed procedures generally occur off-pump. As discussed earlier, in some cases, dissection of tissue is necessary to guide or deliver specialized medical devices to their desired location in the body. In particular, with regard to pulmonary vein antrum isolation, tissue connecting each pair of pulmonary veins to pericardial reflections is often dissected allowing ablation device placement on and/or around the pulmonary veins.

In general, if prior art devices for dissection are used, and if guidance of a specialized medical device to a location after the dissection is desired, separate devices are used for dissection and for placing the specialized medical device. Prior art devices that allow for both dissection and placement of another device, in particular with regard to ablation devices, require suturing a catheter at or near the end of the device while the end of the device is near the heart. Suturing near a beating heart involves risk of negative consequences.

Another challenge to placing ablation devices within or near the heart is that the anatomy of individual patients may differ, requiring different entry points or ports to gain access to the heart. Some current ablation devices include ablating elements connected to rigid elements that are difficult to position within a patient. Manipulation of such rigid elements is problematic and can lead to tissue damage. Also, if a location of an orifice or port does not allow access to a desired part of the heart using such a rigid element, another port must be made in order to reach the desired part.

Ablation devices used for cardiac ablation may have electrodes integrated into jaws of a forceps-like device, which can clamp and ablate tissue between the jaws. Generally the controls for applying ablative energy through the electrodes are located outside the body. Often the controls are located on a generator or switch device that is remote from the handheld portion of the ablation device. Such separate controls may cause the surgeon to direct attention away from the patient. In addition, such separate controls may be out of reach of the surgeon, which means another person may need to manipulate the controls. These issues relating to the proximity of the controls to the surgeon can result in erroneous application of ablative energy at undesired locations in a patient or at undesired times during an ablation procedure. Additionally, with regard to some minimally invasive procedures in particular, such remote controls or switches may be required to be moved around the operating room as the surgeon moves around to access different parts of the body, which is not desired. Even if controls for activating the ablative energy source are located on a handle of the ablation device that is in the hands of the surgeon, during manipulation and placement of the device within a body, the ablative energy controls (e.g., trigger) can be accidentally activated when not desired.

Therefore, there is a need for novel ablation devices, systems for guiding ablation devices into bodies and methods of both using ablation devices and of guiding ablation devices into bodies, which can improve ablation procedures. In particular, the ablation procedures can be improved by decreasing the number of ports necessary to properly access areas of the heart. In addition, ablation procedures may be improved by reducing or eliminating undesired tissue damage such as that caused by using rigid elements to deliver ablating elements. Also, ablation procedures may be improved by avoiding inadvertent application of ablative energy at an undesired location in a body. Further, ablation procedures may be improved by localizing controls to a handle portion that is held by the surgeon.

Some previous ablation devices are described in the following publications U.S. Patent Application Publication No. US 2006/0009759 A1 (Christian et al.); U.S. Patent Application Publication No. US 2006/0036236 A1 (Rothstein et al.); U.S. Patent Application Publication No. US 2006/0020263 A1 (Rothstein et al.); and, U.S. Patent Application Publication No. US 2006/0041254 A1 (Francischelli et al.).

### SUMMARY OF THE INVENTION

The invention is set out in the appended claims. The embodiments and examples of the present description that do not fall within the scope of said claims are provided for illustrative purposes only and do not form part of the present invention.

The present invention relates to ablation of tissue during surgical procedures. The present invention is of particular applicability for use during minimally invasive surgical procedures or endoscopic procedures, such as during ablation procedures on a heart (e.g., pulmonary antrum isolation). The device includes a set of clamping jaws with ablating elements, which are connected to a handle assembly by a flexible neck, with controls for opening and closing the clamping jaws and applying ablative energy controlled remotely in the handle. The flexible neck in the device allows the clamping jaws, and ablating elements, to be easily maneuvered and placed in a desired location in a body. The device also preferably includes a lockout mechanism that prevents the ablative energy from being applied unless the clamping jaws, including the ablating elements, are in a closed position. Preferably, the ablative energy cannot be applied unless the user has deactivated the lockout mechanism. The present invention also preferably includes a system used to guide the ablation device to a location in a body where ablation is desired.

The present invention provides advantages over prior art devices and methods for ablating tissue. One advantage is that the flexible nature of the neck allows the ablation device to fit the anatomies of different patients. Another advantage is that using an ablation device with such a flexible neck can reduce the number of ports of entry into a body that need to be made to perform an ablation procedure, because more areas of the heart may be reached by the device using a single port. Yet another advantage of the present invention is, because the clamping jaws may be in a parallel configuration in a closed position and because the neck is flexible, the jaw end ofthe device may fit easily through small ports used in minimally invasive procedures. A further advantage of the present invention is the flexibility of the neck allows a surgeon to use a variety of approaches to an ablation procedure. An additional advantage is that the clamping jaws are a floating jaw design, which can function with a variety of tissue configurations or thicknesses. A still further advantage is that ablative energy may only be applied when the clamping jaws are in a closed position and the lockout mechanism is deactivated by the user, which avoids applying ablative energy to undesired tissue while maneuvering the device into a body. Further, the controls for the device are conveniently located on the handle, which is being held and controlled by the user. An advantage of the system of the present invention is the option for the ablation device to be able to be rapidly associated and disassociated with a guide wire system to assist in placement of the ablation device.

A first embodiment of the present invention is a device for ablating tissue at a desired location in a body, the device comprising: a pair of floating jaws moveable between a spaced apart open position and a closed position, the pair of jaws comprising at least one ablating element for ablating tissue located between the jaws; a handle comprising controls for remotely controlling the movement of the jaws and the at least one ablative element; and a flexible neck connecting the jaws and handle, wherein the neck is flexible so as to permit the jaws to be maneuverable in the body with respect to the handle. The open position of the jaws in the device may be scissor-like in configuration while that in the closed position may be parallel in configuration. The ablating element of the device may comprise a fluid assisted electrode assembly. The controls of the handle may comprise a lever that when moved causes the jaws to move between the open position and the closed position. The controls for the at least one ablating element may comprise a trigger that activates an ablative power source. The controls of the handle may comprise a lock for locking the jaws in the closed position. The controls of the handle may comprise an overdrive mechanism for limiting closure of the jaws. The overdrive mechanism may comprise a clutch assembly.

A second embodiment is a device for ablating tissue, the device comprising: a pair of floating jaws moveable between a spaced apart open position and a closed position, the pair of jaws comprising at least one ablating element for ablating tissue located between the jaws, wherein the open position of the jaws is scissor-like in configuration and the closed position is parallel in configuration; a handle comprising controls for remotely controlling the movement of the jaws and the at least one ablative element; and a neck connecting the jaws and handle.

A third embodiment of the present invention is a system for guiding an ablation device to a desired location in a body, the system comprising: an ablation device having a pair of jaws comprising at least one ablating element for ablating tissue located between the jaws; and means for guiding the ablation device to the desired location that may be attached and reattached to the ablation device. The ablation device of the system may comprise: a pair of floating jaws moveable between a spaced apart open position and a closed position, the pair of jaws comprising at least one ablating element for ablating tissue located between the jaws; a handle comprising controls for remotely controlling the movement of the jaw and the at least one ablative element; and a flexible neck connecting the jaws and handle, wherein the neck is flexible so as to permit the jaws to be maneuverable in the body with respect to the handle. The means for guiding the ablation device may comprise at least one guide member, the guide member comprising a tube connected to an attachment means that cooperates with an attachment means on the jaws of the ablation device.

### BRIEF DESCRIPTION OF THE DRAWINGS

The present invention will be further explained with reference to the appended Figures, wherein like structure is referred to by like numerals throughout the several views, and wherein:
Fig. 1 is a plan view of an ablation system, in accordance with the present invention, showing an ablation device, first and second guide members, and a guide member adapter;
Fig. 2 is a plan view of an embodiment of a portion of a jaw assembly portion of an ablation device, in accordance with the present invention;
Fig. 3 is a top view of an embodiment of a jaw assembly portion of an ablation device, in accordance with the present invention, showing the jaw assembly in an open position and with a nose component and a spring sleeve retainer component shown in wire frame or phantom;
Fig. 4 is the same jaw assembly as in Fig. 3 except showing the jaw assembly in a more closed position than Fig. 3, and with jaws parallel to each other;
Fig. 5 is a plan view of a jaw assembly portion, a neck portion and power and fluid delivery conduits connected to the jaw assembly portion, in accordance with the present invention;
Fig. 6 is an exploded view of Fig. 5;
Fig. 7 is a close-up view of the jaw assembly portion of Fig. 6;
Fig. 8 is a plan view of an embodiment of a portion of a handle portion of an ablation device, in accordance with the present invention, shown with one of two halves of a handle casing removed to expose components inside the handle, and with a pull wire extending proximally into the handle;
Fig. 9 is a side view of an embodiment of a handle potion of an ablation device, in accordance with the present invention, shown with one of two halves of a handle casing removed to expose components inside the handle, and with a neck attached to the handle;
Fig. 10 is a side view of a clutch assembly, in accordance with the present invention;
Fig. 11 is a plan view ofthe clutch assembly of Fig. 10;
Fig. 12 is an exploded view of the clutch assembly of Figs. 10 and 11;
Fig. 13 is another exploded view of the clutch assembly of Figs. 10 and 11 from a different vantage point from Fig. 12;
Fig. 14 is a exploded view a lever portion (and attached components) of a handle assembly, in accordance with the present invention;
Fig. 15 is a plan view of an embodiment of a portion of a handle portion of an ablation device, in accordance with the present invention, shown with one of two halves of a handle casing removed to expose components inside the handle, and with a neck attached to the handle;
Fig. 16 is a plan view of some components of a lockout mechanism, in accordance with the present invention;
Fig. 17 is another plan view of the same components of the lockout mechanism in Fig. 16 from a different vantage point;
Fig. 18 is an exploded view of the components of the lockout mechanism of Fig. 17;
Fig. 19 is a cross-sectional view of a portion of the handle assembly showing the jaw activation lever in a locked position with the lockout feature deactivated;
Fig. 20 is a cross-sectional view of the same portion of the handle assembly as in Fig. 19, showing jaw activation lever released with the lockout feature activated;
Fig. 21 is a plan view of an embodiment of a portion ofa handle portion of an ablation device, in accordance with the present invention, shown with one of two halves of a handle casing removed to expose components inside the handle, including power wires and fluid delivery conduits;
Fig. 22 is a side view of a cord assembly, in accordance with the present invention;
Fig. 23 is a side view of an embodiment of a jaw assembly portion of an ablation device, in accordance with the present invention, showing curvature of a portion of the jaw assembly comprising clamping jaws;
Fig. 24 is a side view of an embodiment of a jaw assembly portion of an ablation device in accordance with the present invention showing curvature of a portion of the jaw assembly comprising clamping jaws;
Fig. 25 is a plan view of a posterior side of a heart showing two ablation devices closed around the two pairs of pulmonary veins as in an approach to pulmonary antrum isolation resulting in box lesions;
Fig. 26 is a plan view of a posterior side of a heart showing two ablation devices closed around the two pairs of pulmonary veins as in an approach to pulmonary antrum isolation resulting in encircling island lesions;
Fig. 27 is a schematic illustration of a pulmonary vein ostium (not shown in relation to a heart), including a right pair and a left pair of pulmonary veins, with the view being from the anterior side of a body;
Fig. 28 is a schematic illustration of the pulmonary vein ostium of Fig. 27 and showing a step in a method of guiding and using an ablation device, in accordance with the present invention, in which a first guide member is inserted posterior to upper right and left pulmonary veins;
Fig. 29 is a similar view to Fig. 28, showing a subsequent step in the method in which a second guide member is inserted posterior to lower right and left pulmonary veins;
Fig. 30 is a similar view to Fig. 29, showing a subsequent step in the method in which an ablation device, in accordance with the present invention, is shown attached to the first and second guide members;
Fig. 31 is a plan view of a portion of a shroud assembly on a distal end of a clamping jaw of an ablation device, in accordance with the present invention, shown separated from an end portion of a guide member, in accordance with the present invention;
Fig. 32 is a similar view to Fig. 31, showing a step in a method of inserting the end portion of the guide member being into an orifice of the shroud assembly;
Fig. 33 is a similar view to Fig. 32, showing a subsequent step in the method in which the end portion of the guide member is inserted into an orifice of the shroud assembly;
Fig. 34 is a plan view of a shroud assembly on a distal end of a clamping jaw of an ablation device and of an end portion of a guide member, in accordance with the present invention, showing a step in a method of inserting the guide member into the shroud assembly;
Fig. 35 is a similar view to Fig. 30, showing a subsequent step in the method in which the ablation device is pulled into place around the right pair of pulmonary veins;
Fig. 36 is a similar view to Fig. 35, showing a subsequent step in the method in which the ablation device is in an open position after ablation and an ablation lesion is shown;
Fig. 37 is a similar view to Fig. 36, showing a subsequent step in the method in which the ablation device is withdrawn;
Fig. 38 is a top view of a jaw assembly and of an end portion of a guide member, in accordance with the present invention, showing the guide member connected to one clamping jaw of the jaw assembly, and an arrow indicating the direction the guide member be moved for removal from the clamping jaw, which is a step in a method for removing the guide member from the clamping jaw;
Fig. 39 is a similar view to Fig. 38, showing a subsequent step in the method in which the guide member is moved toward the interior of the clamping jaws in order to remove the guide member;
Fig. 40 is a similar view to Fig. 39, showing a subsequent step in the method in which the guide member is removed from the clamping jaw;
Fig. 41 is a similar view to Fig. 40, showing a subsequent step in the method in which the ablation device is removed from the guide members;
Fig. 42 is a similar view to Fig. 41, showing a subsequent step in the method in which the ablation device attached to the two guide members on the opposite ends from a prior step;
Fig. 43 is a similar view to Fig. 42, showing a subsequent step in the method in which the ablation device is pulled into place for ablation surrounding the left pair of pulmonary veins;
Fig. 44 is a similar view to Fig. 43, showing a subsequent step in the method in which the ablation device is in an open position after ablation and an ablation lesion is shown;
Fig. 45 is a similar view to Fig. 44, showing a subsequent step in the method in which the ablation device is withdrawn;
Fig. 46 is a similar view to Fig. 45, showing the resulting pulmonary ostium, with two ablation lesions, after the previous steps in the method;
Fig. 47 is a schematic illustration of a pulmonary vein ostium (not shown in relation to a heart), including a right pair and a left pair of pulmonary veins, with the view being from the anterior side of a body, showing a step in a method in which a dissector/guide is placed with a distal end surrounding the right pair of pulmonary veins;
Fig. 48 is a plan view of an end portion of a guide member being inserted into a guide member adapter, in accordance with the present invention, as indicated by arrow;
Fig. 49 is a plan view of a guide member connected to a guide member adapter, in accordance with the present invention;
Fig. 50 is a similar view to Fig. 49, showing a subsequent step in the method in which a guide member with attached guide member adapter is shown attached to the distal end of the dissector/guide;
Fig. 51 is a similar view to Fig. 50, showing a subsequent step in the method in which the dissector/guide is withdrawn and pulls the guide member to surround the right pair of pulmonary veins;
Fig. 52 is a similar view to 51, showing a subsequent step in the method in which the dissector/guide is removed from the guide member; '
Fig. 53 is a similar view to Fig. 52, showing a subsequent step in the method in which an ablation device, in accordance with the present invention, is attached to the guide member;
Fig. 54 is a similar view to Fig. 53, showing a subsequent step in the method in which the ablation device is pulled into place for ablation surrounding the right pair of pulmonary veins;
Fig. 55 is a similar view to Fig. 54, showing a subsequent step in the method in which the ablation device is in an open position after ablation and an ablation lesion is shown;
Fig. 56 is a similar view to Fig. 55, showing a subsequent step in the method in which the guide member and the ablation device are withdrawn;
Fig. 57 is a similar view to Fig. 56, showing a subsequent step in a method in which the dissector/guide is placed with the distal end surrounding the left pair of pulmonary veins;
Fig. 58 is a similar view to Fig. 57, showing a subsequent step in the method in which a guide member with attached guide member adapter is shown attached to the distal end of the dissector/guide;
Fig. 59 is a similar view to Fig. 58, showing a subsequent step in the method in which the dissector/guide is withdrawn and pulls the guide member to surround the left pair of pulmonary veins;
Fig. 60 is a similar view to Fig. 59, showing a subsequent step in the method in which the dissector/guide is removed from the guide member;
Fig. 61 is a similar view to Fig. 60, showing a subsequent step in the method in which an ablation device, in accordance with the present invention, is attached to the guide member;
Fig. 62 is a similar view to Fig. 61, showing a subsequent step in the method in which the ablation device is pulled into place for ablation surrounding the left pair of pulmonary veins;
Fig. 63 is a similar view to Fig. 62, showing a subsequent step in the method in which the ablation device is in an open position after ablation and an ablation lesion is shown; and
Fig. 64 is a similar view to Fig. 63, showing a subsequent step in the method in which the guide member and the ablation device are withdrawn.

### DESCRIPTION OF THE PREFERRED EMBODIMENT

In the following detailed description of the preferred embodiments, reference is made to the accompanying Figures which form a part hereof, and in which is shown by way of illustration specific embodiments in which the invention may be practiced. It is to be understood that other embodiments may be utilized and structural or logical changes may be made without departing from the scope of the present invention. The following detailed description, therefore, is not to be taken in a limiting sense, and the scope of the present invention is defined by the appended claims.

With reference to the accompanying Figures, wherein like components are labeled with like numerals throughout the several Figures, ablation devices, ablation systems, and methods of use thereof are disclosed, taught and suggested by the multiple embodiments for the purpose of ablation of tissue in a subject body. It is understood that any of the ablation devices, systems and methods, in accordance with the present invention, have applicability for use in any part of a subject's body, including the human body or other animals or creatures, where ablation is useful. The present invention is described below as developed for the application of ablation of cardiac tissue, and in particular for pulmonary vein antrum isolation, in the treatment of atrial fibrillation, as described above in the Background section. However, it is contemplated that the ablation devices, systems and methods may be used for treating any condition for which ablation of tissue is useful.

A device contemplated by the present invention preferably includes basic functionality for ablating tissue in a location in a body. Such a device preferably includes a manner of allowing clamping jaws, and included ablating elements, to be easily maneuvered and placed in a desired location in a body. In addition, such a device preferably includes a manner of preventing ablative energy from being applied unless the clamping jaws, including the ablating elements, are in a closed position and the user has deactivated a mechanism that deactivates an ablative energy source. Also, such a device preferably includes controls that are in close proximity to the user, and more preferably on a handheld portion of the device. Still further, such a device may be part of a system for guiding the device to a location in a body. Such a system preferably includes a manner of attaching, detaching and possibly reattaching at least one guide member to the ablation device in order to assist in guiding the ablation device to a desired location in a body.

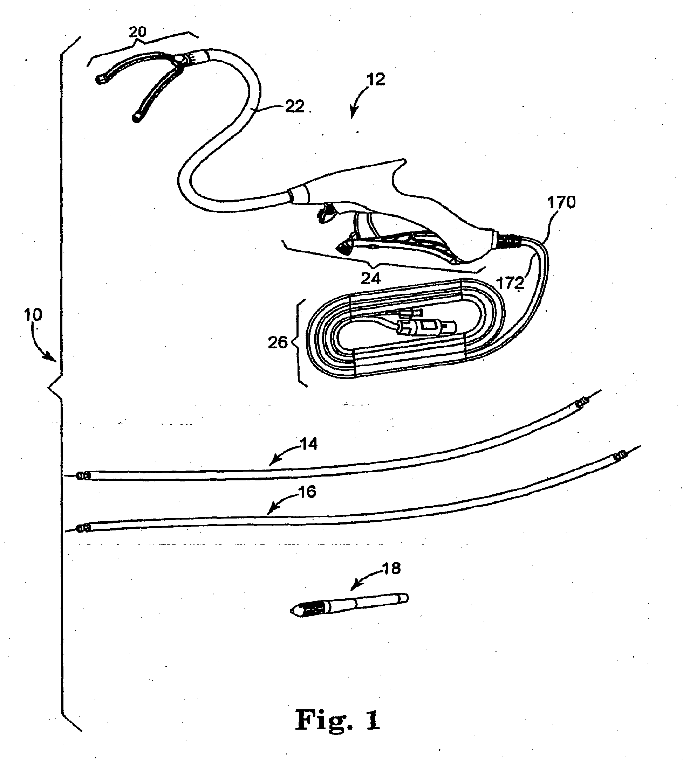

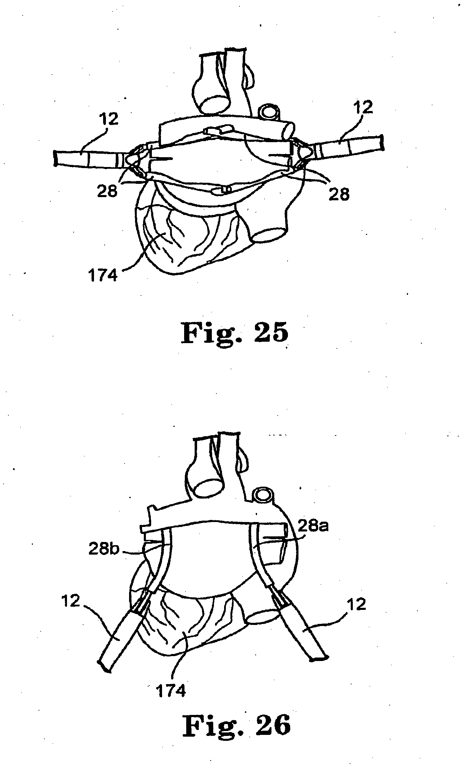

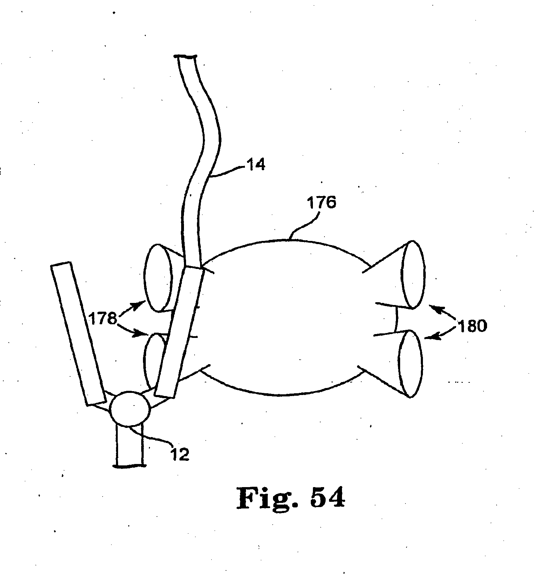

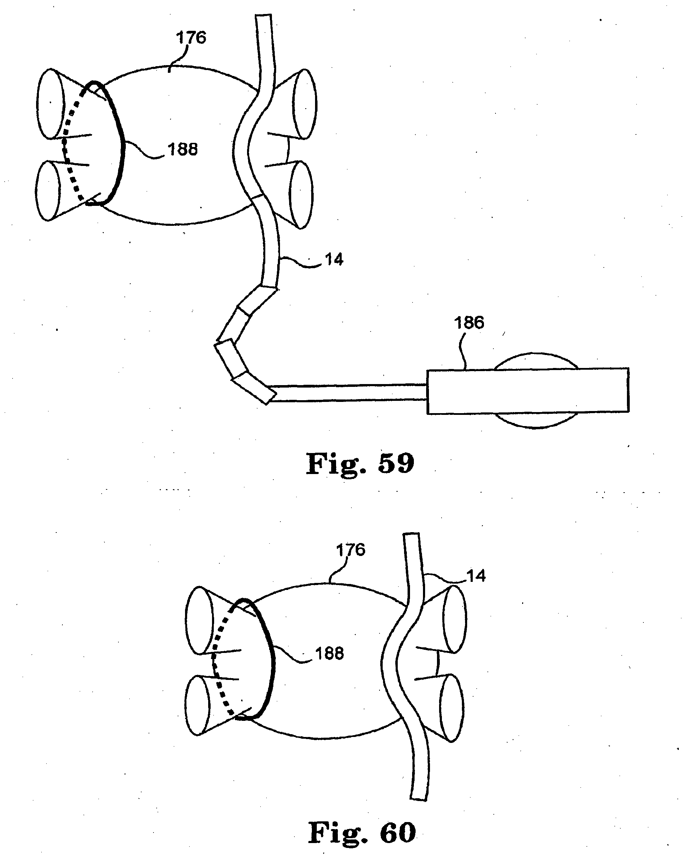

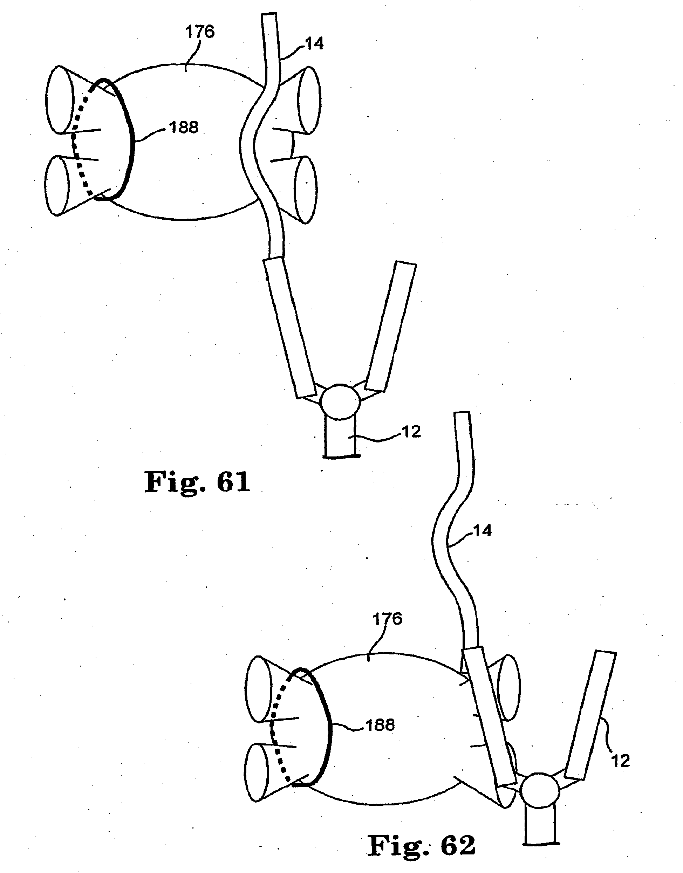

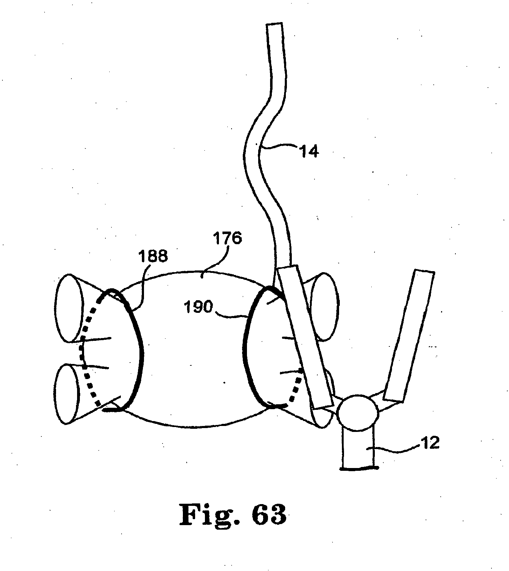

With reference initially to Fig. 1, an exemplary ablation system 10, including an exemplary ablation device 12, is illustrated. The ablation system 10 also comprises at least one guide member (shown with first and second guide members 14, 16) and, optionally, a guide member adapter 18. The ablation device 12 may be used alone or with one or both of the guide members 14, 16, which may attach or connect to the ablation device 12 and may pull the ablation device 12 into a desired position where ablation may take place. The guide members 14, 16 may also preferably be able to be attached or connected to an ablation device, or other device, detached from the device and then reattached. The guide member adapter 18 shown may be used and attached to one of the guide members 14 or 16 in order to allow a guide device to guide or place the ablation device 12 in order to perform an ablation procedure (e.g., as shown in Figs. 26, 47 - 66, and described below).

The exemplary embodiment of the ablation device 12 shown in Fig. 1 generally comprises: a jaw assembly 20, a flexible neck 22 connecting the jaw assembly 20 to a handle assembly 24, and a cord assembly 26 attached to the handle assembly 24. Each of the general portions of the ablation device 12, and its components, will be discussed in detail below.

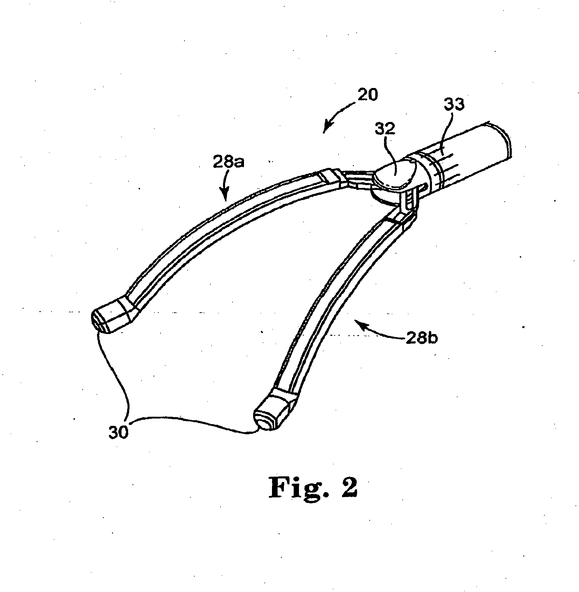

In order to ablate desired tissue, the tissue is retained or clamped using the jaw assembly 20 of the ablation device 12 prior to ablation. Fig. 2 illustrates a plan view of a portion of the jaw assembly 20. The portion of the jaw assembly 20 shown includes a pair of clamping jaws (right 28a and left 28b) that are primarily mirror images of each other, and when in a closed position allow the jaw assembly 20 to clamp tissue. Fig. 2 also includes a shroud assembly 30 on the distal end of each jaw 28a, 28b, which provide a means for attaching and detaching a guide member to and from, respectively, the distal end of each jaw 28a, 28b (the details of the guide member will be discussed below). Additionally, Fig. 2 includes a nose 32 in which the proximal ends of the jaws 28a, 28b, and other components used to open and close the jaws 28a, 28b, are housed and assembled. Also in Fig. 2, a jaw return spring sleeve 33 is shown positioned over a portion of the nose 32.

Fig. 2 also illustrates the jaws 28a, 28b as being preferably curved, other shapes are also possible. The purpose of the curvature illustrated in Fig. 2 is to allow the jaws 28a, 28b to fit around certain anatomical features, such as blood vessels, and to clamp tissue in a desired location with respect to such anatomical features.

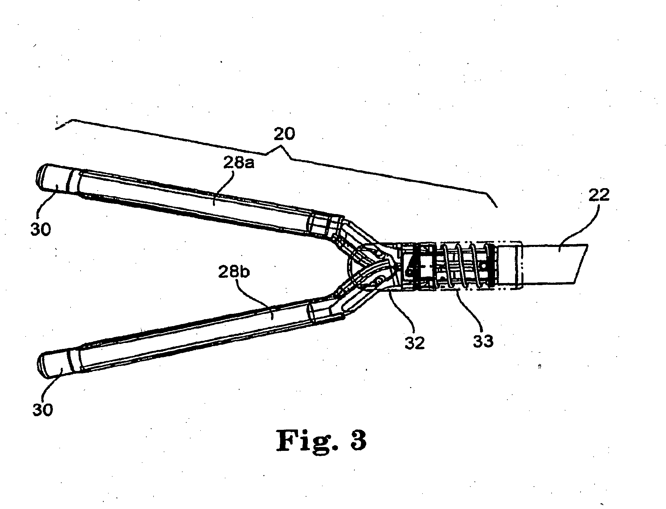

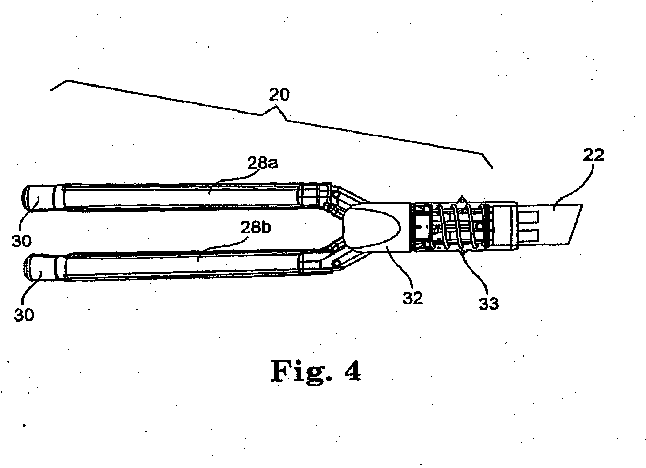

In order to clamp and release tissue, the jaws 28a, 28b of the jaw assembly 20 preferably move between an open position (as seen in Figs. 1-3) and a closed position. So as to move between the open and closed positions, the jaw assembly 20 preferably includes components as depicted in Fig. 3. Fig. 3 illustrates a top view of a preferred embodiment of the jaw assembly 20, shown with the nose 32 and jaw return spring sleeve 33 in wire frame or phantom. Preferably, the components of the jaw assembly 20 are configured so that as the jaws 28a, 28b begin to close, the movement is pivotal or scissor-like, but as the jaws 28a, 28b move closer to each other, the jaws 28a, 28b ultimately close in a parallel configuration, as shown in the partially closed position ofthe jaws 28a, 28b in Fig. 4. By "scissor-like" it is meant that the orientation of the jaws is angular with respect to each other as if from a pivot point when the jaws 28a, 28b are in a generally open position. As the jaws 28a, 28b begin to be closed and continue to move toward one another, they pivot with respect to one another much like a scissor moves until they reach a certain point at which they move parallel to one another. By having the jaws 28a, 28b ultimately come together in a generally parallel configuration, substantially all of the tissue-contacting side surface of the jaws 28a, 28b comes into contact with tissue at about the same time and may exert a more even force on the tissue along the length of the tissue-contacting side surface of the jaws 28a, 28b. Also, the jaws 28a, 28b are preferably able to float to a limited degree with respect to one another as they close or as they are closed together to facilitate contact with uneven tissue surfaces as will also be further described below.

A purpose of the jaws 28a, 28b being moveable and being able to both close (i.e., approximate) and open is to clamp and release tissue to be ablated, as discussed above. However, another purpose of the approximating jaws 20 is to allow the jaw assembly 20, while in a substantially closed position, to be sized and shaped to be able to pass through a 12 mm or other size of trocar port in a patient during minimally invasive surgery.

The jaw assembly 20 is preferably configured such that the jaws 28a, 28b are able to compensate for a variation in tissue configurations or thicknesses. The design of the jaw assembly 20 is preferably configured so that the jaws 28a, 28b close in an independently floating fashion. In particular, the floating jaw assembly 20 permits tissue of varying thicknesses to be clamped in the jaws 28a, 28b with the jaws 28a, 28b coming into contact with tissue generally along their lengths. For example, thicker tissue can be located closer to the nose 32 than thinner tissue, and the jaws 28a, 28b will not be held open by the thick tissue, but will close and contact tissue along their lengths.

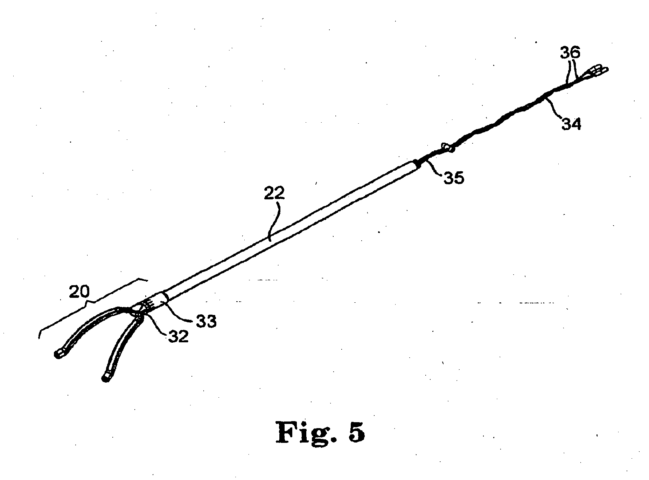

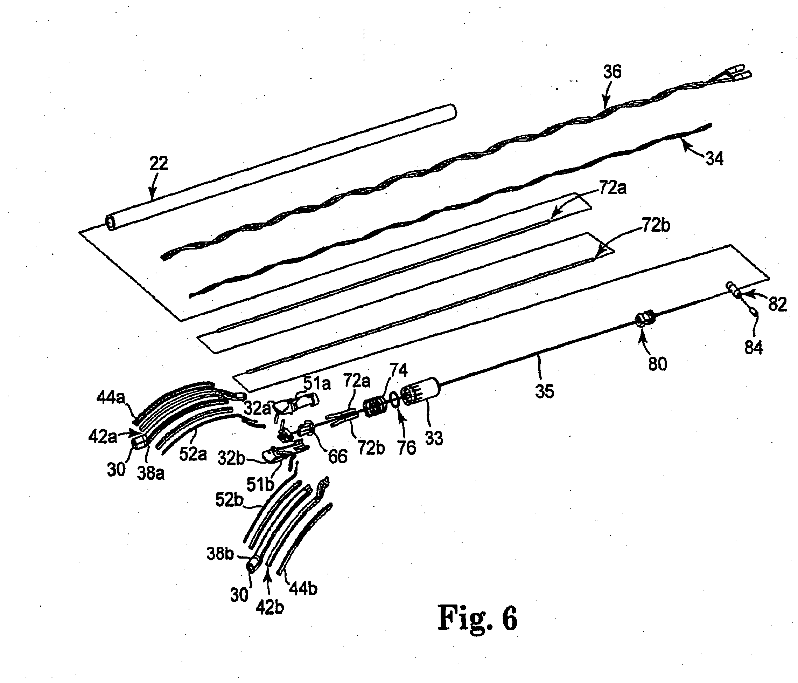

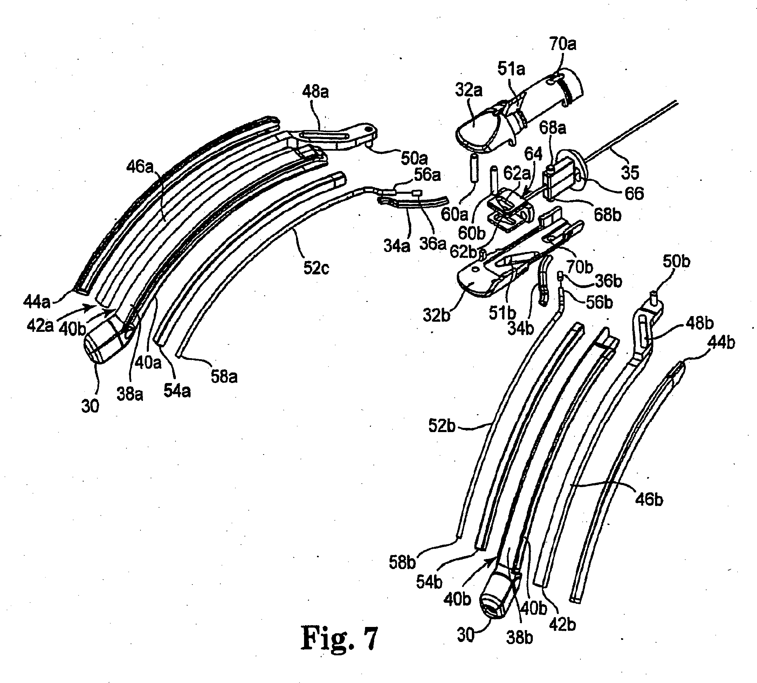

Controls for clamping and ablating tissue are located remotely from the jaws 28a, 28b and are preferably located in the handle assembly 24 that may preferably be handheld. Fig. 5 shows components that extend distally from the handle assembly 24, through the neck 22 and to the jaw assembly 20 in order to control clamping and ablation in the jaw assembly 20, as well as the components of the jaw assembly 20 and neck 22. In the exemplary embodiment shown in Fig. 5, components that extend to the jaw assembly 20 through the neck 22 from the handle assembly 24, are two power source wires 34 (e.g., radiofrequency (RF) wires) intertwined with two fluid delivery conduits 36 (e.g., saline delivery tubes), and a pull wire 35. Fig. 6 is an exploded view of all the components ofthe portion ofthe preferred ablation device 12 shown in Fig. 5. Fig. 7 is a close-up view of a substantial amount of the exploded jaw assembly 20 shown in Fig. 6. Referring to Figs. 5-7, the components of the preferred embodiment shown will be described below. However, it should be noted that the described embodiment is preferred and other variations including ablation devices powered and/or controlled in other ways as known or developed that may include some of the components discussed and/or additional components not discussed are also contemplated by the present invention.

Referring to Figs. 5-7, and beginning with the jaw assembly 10, the preferred jaw assembly 20 of the present invention includes two jaws 28a, 28b with each jaw 28a, 28b including a housing 38a, 38b (respectively). The purpose ofthe housing 38a, 38b is to house the components necessary to approximate the jaws 28a, 28b and to ablate tissue (which will be discussed below). The housings 38a, 38b are preferably made of an electrically insulating material, and include at least two channels, each that run lengthwise, with a first channel 40a on each jaw 28a, 28b facing each other so as to contact tissue between them and a second channel 40b in each housing 38a, 38b facing oppositely. Jaw arms 42a, 42b are provided as to fit into the second channels 40b in the jaw housings 38a, 38b. The jaw arms 42a, 42b are shown retained in the housings 38a, 38b by electrically insulated covers 44a, 44b that are held in place in the housings 38a, 38b. The jaw arms 42a, 42b are controllably moveable and are operatively connected with the housings 38a, 38b and attached to other jaw assembly 20 components in order to provide controlled movement to the jaws 28a, 28b. The jaw arms 42a, 42b include elongate portions 46a, 46b that are retained in the second channels 40b of the respective jaw housings 38a, 38b. Also, as seen in Fig. 7, the jaw arms 42a, 42b preferably include slots 48a, 48b that are proximal to the elongate portions 46a, 46b and that angle towards the interior of the jaw assembly 20, or tissue-contacting side of the jaws 28a, 28b, as the slots 48a, 48b extend proximally. The jaw arms 42a, 42b also each preferably include a pin 50a, 50b on the proximal end of each respective jaw arm 42a, 42b, with the pin 50a extending downward on the right arm 42a and the pin 50b extending upward on the left jaw arm 42b in the illustrated orientation. The slots 48a, 48b and pins 50a, 50b of the jaw arms 42a, 42b cooperate with other components in the jaw assembly 20, which will be discussed below, in order to open and close the jaws 28a, 28b.

In order to ablate tissue, a fluid assisted elongate electrode assembly is preferably provided in the channel 40a in each housing 38a, 38b. The electrode assembly preferably comprises an elongate tubular electrode 52a, 52b that is retained in the channel 40a and as such are preferably provided within lumens of porous electrode supports 54a, 54b. Preferably, the elongate tubular electrodes 52a, 52b include a series of fluid ports (not seen in Figs.) that are open from an internal fluid passage (not shown) and oriented toward the tissue-contacting side of each jaw 28a, 28b so that a conductive fluid may be dispensed from the electrodes 52a, 52b through the series of fluid ports then migrate laterally through the pores of the porous electrode support 54a, 54b and around its circumference to thoroughly and uniformly wet the porous electrode support 54a, 54b along the right and left jaws 28a, 28b. The conductive fluid (e.g., saline) is preferably provided to each of the electrodes 52a, 52b through separate fluid delivery conduits 36a, 36b (only end portions of the fluid delivery conduits 36a, 36b are shown in Fig. 7).

The elongate tubular electrodes 52a, 52b are preferably formed of thin-walled, malleable stainless steel tubing extending between a proximal open end 56a, 56b and a distal, closed end 58a, 58b. The series of fluid ports are formed, e.g., laser drilling, though the sidewall ofthe tubing from a lumen inside and preferably extend in a single line, although the fluid ports could be formed in any selected array extending around the circumference of the sidewall of the tubing. The electrode supports 54a, 54b preferably comprise a porous polymer such as Porex™ plastic.

The elongate tubular electrodes 52a, 52b are flat electrodes that are preferred because the flat design allows for more energy to be applied to the surface of tissue to be ablated. However, other types and shapes of electrodes or ablating elements are also contemplated by the present invention. Other possible ablating elements are energy transfer elements that transfer energy to target tissue. For example, energy may be conductive elements that may supply RF energy (as shown in Figs), HIFU energy, microwave energy, thermal energy, cryogenic energy or ultrasound energy to target tissue. Energy transfer elements may be, for example, laser elements for supplying laser light to target tissue. Two or more energy transfer elements or conductive elements may be arranged in a bipolar arrangement (as shown in Figs.) wherein at least one element is used as a positive electrode and at least one element is used as a negative electrode. One or more energy transfer elements or conductive elements of the ablation device 12 may be arranged in a monopolar arrangement wherein at least one element is used as one electrode and an indifferent electrode is placed elsewhere on the patient's body such as the back, thigh or shoulder or another site other than the ablation device 12 site.

Energy transfer elements or conductive elements may comprise one or more conductive materials or blends including titanium, titanium alloys, TiNi alloys, shape memory alloys, super elastic alloys, aluminum oxide, platinum, platinum alloys, stainless steels, stainless steel alloys, MP35N, elgiloy, haynes 25, satellite, pyrolytic carbon, silver carbon, conductive metals, conductive polymers or plastics, and/or conductive ceramics. Energy transfer elements or conductive elements may not be conductive but may serve as a conduit to deliver a conductive material such as a conductive fluid. Energy transfer or conductive elements may be porous. For example, energy transfer elements or conductive elements may comprise porous polymers, metals, or ceramics. Energy transfer elements or conductive elements may be coated with non-stick coatings such as PTFE or other types of coatings as discussed herein. In particular, the energy transfer elements may comprise one or more coatings, e.g., hydrophilic coatings. Energy transfer elements or, conductive elements may be flexible thereby allowing them to conform to the surface of target tissue. Energy transfer elements or conductive elements may be malleable thereby allowing a surgeon to shape them to conform to the surface of target tissue.

Energy transfer elements or conductive elements may comprise one or more metal conductors such as windings inside a polymer or a conductive mesh material. The energy transfer elements or conductive elements may comprise tubes for delivery of fluids. The tubes may comprise holes or slots. A polymer tube may be placed inside a metal tube to control fluid delivery through energy transfer elements or conductive elements. One or more of the energy transfer elements or conductive elements may be used as one or more nerve stimulation electrodes and/or as one or more cardiac stimulation electrodes. Electrodes may be used for cardiac pacing, defibrillation, cardioversion, sensing, stimulation and/or mapping.

Energy transfer elements or conductive elements may comprise needles designed to penetrate tissues such as fat and muscle. For example, energy transfer elements or conductive elements may be designed to penetrate fat on the heart thereby allowing the energy transfer elements or conductive elements to reach cardiac tissue. The needles may allow fluids such as conductive fluids, chemicals such as ablation chemicals, drugs, biological agents and/or cells to pass through. The needles may allow a vacuum or suction to pass through.

In additional embodiments, the ablation device 12 of the present invention may include means for tracking the position of the ablation device 12. The means for tracking the position of the ablation device 12 may include, for example, sensors and imaging devices. An example of a disclosure of such a tracking means is described in U.S. Patent Application Publication US 2006/0229594 A1 (Francischelli et al.).

Adhesive may be applied to maintain the elongate tubular electrodes 52a, 52b and porous electrode supports 54a, 54b in the channels 40a in the jaw housings 38a, 38b. The adhesive used may not block migration of conductive fluid around the porous electrode supports 54a, 54b.

In order to supply energy or power to the elongate tubular electrodes 52a, 52b, power source wires 34, in the preferred embodiment, extend distally from a power source (preferably separate from ablation device 12) through the neck 22 and are soldered to the elongate tubular electrodes 52a, 52b, for example, as shown in Fig. 7 (only portions of wires 34 shown in Fig. 7), which is preferably at a location where the electrodes 52a, 52b are not surrounded by electrode supports 54a, 54b.

Other methods of irrigating the electrodes or ablating elements, besides that method described above, are also contemplated by the present invention. The purpose of irrigation of the electrodes with saline or other conductive fluid is to help decrease the interface impedance, cool the tissue, and allow for a greater lesion depth. Irrigation can also help prevent tissue or fat from clogging the electrodes and help keep the electrodes clean.

Figs. 6 and 7 show other components that cooperate with the jaw arms 42a, 42b in order to approximate the jaws 28a, 28b. The figures illustrate two halves 32a, 32b of the nose 32. The two halves, as shown, preferably have the same shape and are made to mate or connect together as shown, and house components used for approximation. Two identical pins 60a, 60b are disposed, as shown in Fig. 7, between and attached to the two halves 32a, 32b of the nose 32. The slot 48a on jaw arm 42a is slidably retained on pin 60a and slot 48b is slidably retained on jaw arm 42b. The pins 50a, 50b on the jaw arms 42a, 42b are moveably retained in triangular-shaped openings 62a, 62b on the top and bottom of a clevis 64 that is moveably retained in the nose 32. The pins 50a, 50b on the jaw arms 42a, 42b are also moveably retained in openings 51a, 51b in the nose halves 32a, 32b. The clevis 64, at its proximal end, is attached to the pull wire 35. From the clevis 64, the pull wire 35 extends proximally through a distal neck retainer barb 66, which is attached to the nose halves 32a, 32b by extensions 68a, 68b on the distal neck retainer barb 66 as being fitted within apertures 70a, 70b on the nose halves 32a, 32b. The purpose of the distal neck retainer barb 66 is to attach the neck 22 to the nose 32 so that the pull wire 35 moves relative to the neck 22 and nose 32 as they are operatively fixed together.

In order to close the jaws 28a, 28b while in an open position, the pull wire 35 is pulled from the proximal portion of the device 12 (how this is performed is discussed in more detail below with regard to the handle portion 24), which results in the clevis 64 moving proximally within a formed interior cavity of the nose 32. As the clevis 64 is pulled proximally, it exerts force on the jaw arms 42a, 42b, which are connected to the clevis 64 by the pins 50a, 50b. As the jaw arms 42a, 42b are pulled proximally for an initial distance within the nose 32, the slots 48a, 48b slide along the pins 60a, 60b in the nose 32, which moves the jaws 28a, 28b toward each other in a scissor-like motion with the pins 60a, 60b located at an intermediate point within the slots 48a, 48b. At that point, the jaws 28a, 28b are preferably substantially parallel as controlled by the shape of the slots 48a, 48b and interaction with the pins 60a, 60b. Once the jaws 28a, 28b are substantially parallel (but not yet closed), further pulling proximally on the clevis 64 pulls the jaws 28a, 28b further proximally as well. The pins 50a, 50b are extending through the slots 62a, 62b in the clevis 64 are guided through the slots 51a, 51b in the nose halves 32a, 32b. The shape of slots 51a, 51b force the pins 50a, 50b and thus the jaws 28a, 28b to move toward each other as the pull wire 35 is further moved proximally relative to the neck 22 and nose 32. At the same time, the width of slots 62a, 62b of the clevis 64 permit inward movement of pins 50a, 50b. Also, pins 60a, 60b slide along slots 48a, 48b. The combination of interactions between pins 50a, 50b and 60a, 60b, and slots 48a, 48b and 51a, 51b results in the jaws 28a, 28b moving toward each other in a substantially parallel position until the jaws 28a, 28b are in a substantially closed position (contacting each other). The slots 51a, 51b also limit how far the clevis 64 may move proximally in the nose 32. This arrangement of pins and slots also permits the jaws 28a, 28b to float to the degree permitted by the interaction of the pins arid slots so that the jaws 28a, 29b can adjust in orientation relative to one another based upon counter-pressure applied to the jaws surfaces from the engagement with tissue.

The pull wire 35 extends from the handle 24 portion through the neck 22 and into the jaw assembly 20 through a lumen in the distal neck retainer barb 66. Fig. 6 shows that the pull wire 35 is surrounded by an incompressible coil 72b, which is then further surrounded by a sleeve 72a. A preferred material for the sleeve 72a is polyimide, although other materials are also contemplated. The purpose of such a sleeve 72a is to protect the pull wire 35 as it is pulled through parts of the jaw assembly 20, and also as the components are bent and moved around in the flexible neck 22. Preferably, as shown in Fig. 6, the power source wires 34 and fluid delivery conduits 36 are also spirally wound through the neck 22 for strain relief.

In order to return the jaws 28a, 28b from a closed position to an open position, the jaw assembly 20 includes a jaw return spring 74 (see Fig. 6) (which happens when no tension is placed on the pull wire 35). Fig. 6 also shows that the jaw return spring 74 is preferably held in place surrounding the nose 32 at its proximal end by a retaining ring 76 that provides bias between the end of the nose 32 and the clevis 64 to move the clevis 64 distally. The spring 74 is provided in contact with the clevis 64 and exerts force in a distal direction on the clevis 64 in order to return the jaws arms 42a, 42b to an open position. Also shown in Fig. 6, is a jaw return spring sleeve 78 that covers the jaw return spring 74 and retaining ring 76. Other biasing arrangements with other components and/or configurations that would also return the jaws 28a, 28b to an open position are also contemplated by the present invention.

The pull wire 35 extends proximally in the device 12 from the jaw assembly 20, through the neck 22 and into the handle 24. As the pull wire 35 enters the handle 24, the pull wire 35 is fed through a proximal neck retainer barb 80 (shown on Fig. 6), which attaches the neck 22 to the handle assembly 24, and the pull wire 35 continues into the handle assembly 24 and attaches at its distal end to a wire terminal 82 (also shown on Fig. 6). The wire terminal 82 is held in place in the handle 24 using a set screw 84 (Fig. 6).

The pull wire 35 is preferably made of stainless steel, although other suitable materials may be used, with a solid wound coil surrounding the pull wire 35. The preferred configuration of the pull wire 35 and surrounding coil is an incompressible coil. Other suitable materials and/or designs that act as an incompressible coil are also contemplated by the present invention. A purpose of the incompressible coil configuration is to maintain the overall length of the pull wire 35 when the portion of the pull wire 35 that extends through the flexible neck 22 is flexed or twisted etc.

The jaw assembly 20 is functionally connected to the handle assembly 24 by the neck 22. A purpose of the neck 22 is to provide a shaft or lumen through which components (e.g., power source wires 34, fluid delivery conduits 36 and pull wire 35) may extend between the jaw assembly 24 and the handle assembly 24. The length of the neck 22 then is preferably related to the distance required in a procedure to allow the jaw assembly 20 to be at a desired anatomical location with the handle assembly 24 being outside the body (i.e., *ex vivo*).

The neck 22, which attaches the jaw assembly 20 to the handle 24, is flexible or "floppy" in nature. In one embodiment, the neck 22 may be flexible or floppy like a rope, for example. The flexible or "floppy" nature may thereby allow a guide member or device to be used to easily position the jaw assembly 20 ofthe ablation device 12 into a position to ablate tissue. The flexible nature of the neck 22 enables the ablation device 12 to be used with many different anatomies found in different patients. The neck 22 may be capable of effectively transmitting torque.

Preferably, the neck 22 is made of extruded polyurethane with a 304 stainless steel braid. However, other suitable components or designs that provide the desired flexibility of the neck 22 are also contemplated by the present invention.

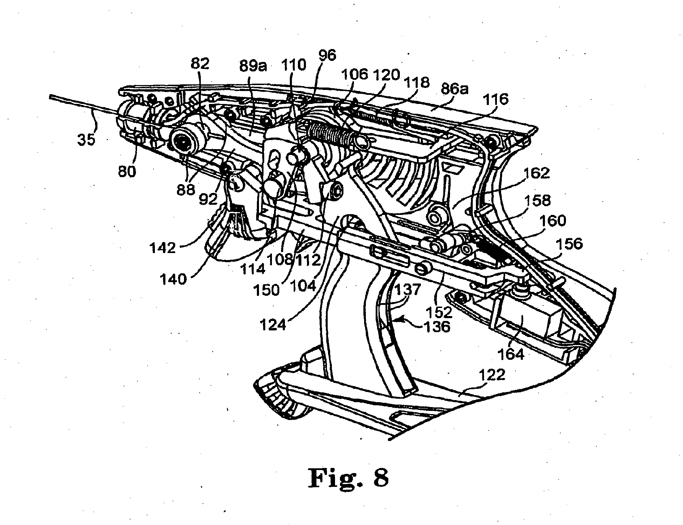

In order to control approximation of the jaws 28a, 28b and application of ablative energy, which both take place at or near the jaw assembly 20 of the ablation device 12 preferably when the jaw assembly 20 is placed at a desired location in a body, the controls for approximation and ablation are preferably located *ex vivo*. Preferably, the controls are located in and/or on the handle assembly 24, which remains *ex vivo* during an ablation procedure. Preferably, the handle assembly 24 comprises a handle casing 86 having two mating handle casing halves (one half of which is shown in Fig. 8 as 86a) for housing the other components and for providing a hand piece for the user of the device. Also, preferably, the handle assembly 24 may be held in the hand of a user.

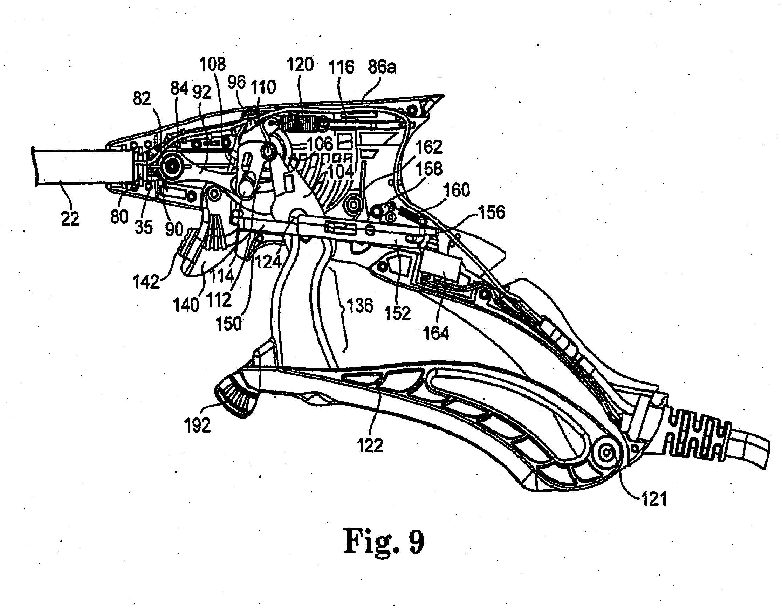

As discussed previously, in order to cause the components of the jaw assembly 20 to close the jaws 28a, 28b, the pull wire 35 is pulled proximally using controls in the handle assembly 24. Referring to Figs. 8 and 9, in general, in order to pull the pull wire 35 proximally, a jaw activation lever 122 is squeezed or moved toward the handle casing 86a (only one half shown) by the user, which results in coordinated and controlled movement of various linked components that work together to pull the pull wire 35 proximally. The handle assembly 24 also includes components that enable the pull wire 35 to be held in the proximal position and that enable the movement of the components to be reversed to allow for release of the pull wire 35 and opening of the jaws 28a, 28b.

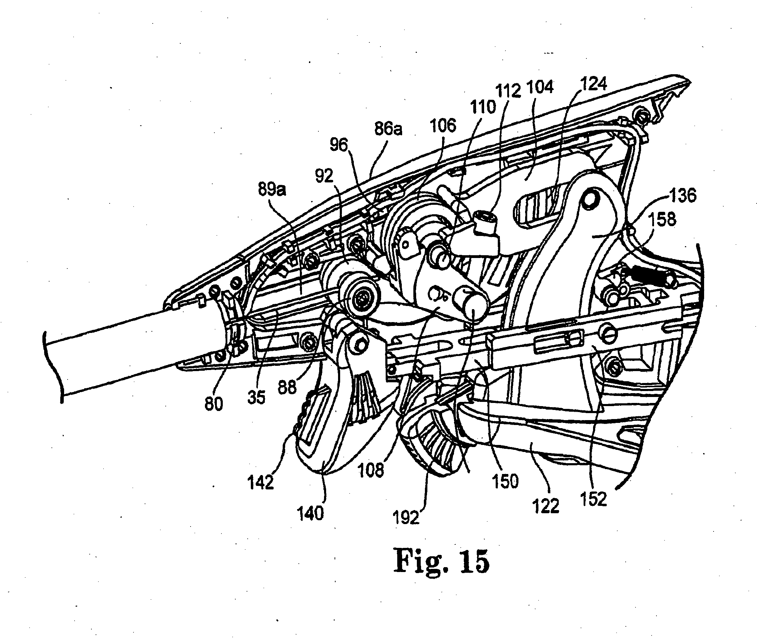

In the preferred embodiment shown in the figures, and in Figs. 8 and 9 in particular, the pull wire 35 extends from the neck 22 into the handle casing 86 through the proximal neck retainer barb 80, and is connected to the wire terminal 82. Preferably, the wire terminal 82 is held in place with the set screw 84. The ends of the wire terminal 82 are preferably attached to two rollers 88 that are retained in recesses (one recess in the handle housing half 86a, seen in Figs. 8, 15 as 89a) in both halves 86a, 86b (not shown) ofthe handle casing 86, which allow the rollers 88 to rotate and provide predetermined paths for the rollers 88. The wire terminal 82 is also placed through an aperture 90 in a distal end of a link arm 92, with the aperture 90 being sized and shaped to retain the wire terminal 82.

In general, a basic purpose of the clutch assembly 94 is to translate the motion of the jaw activation lever 122, both toward and away from the handle casing 86, into generally proximal and distal, respectively, motion of the link arm 92. The link arm 92, in turn, moves the pull wire 35 proximally or distally, which closes or opens the jaws 28a, 28b, respectively.

The clutch assembly 94, as shown in Figs. 8-13, generally preferably includes the link arm 92 that is connected to the pull wire 35 and which is attached to other components of the clutch assembly 94 that pivot around an axle 110 and that are attached to a cam 104 that may be rotated by movement of the jaw activation lever 122. The clutch assembly also preferably includes components that generally allow overdrive slip (i.e., components that comprise an overdrive mechanism) so that, for example, once the jaws 28a, 28b are closed around tissue with a certain force, the jaw activation lever 122 may continue to be squeezed toward the handle casing 86 and the cam 104 rotated in order to, for example, lock the lever 122 in place, without additional proximal pulling on the pull wire 35 nor further approximation of the jaws 28a, 28b. The clutch assembly 94 also preferably includes a tension adjuster mechanism by which to adjust the tension in the overdrive slip to accommodate different thicknesses of tissue to be ablated, for example.

More particularly, with regard to the components of the clutch assembly 94, in order to close the jaws 28a, 28b, the pull wire 35 is pulled proximally as the wire terminal 82 is pulled proximally in the recesses (one of which is 89a) by the link arm 92. The purpose of allowing the rollers 88 and attached wire terminal 82 to rotate in the recesses (one of which is 89a), while the link arm 92 of the clutch assembly 94 moves generally proximally, is to prevent bending the pull wire 35 in the handle assembly 24, which could in turn cause tension and fracture the pull wire 35 as it extends out through the neck 22 and into the jaw assembly 20.

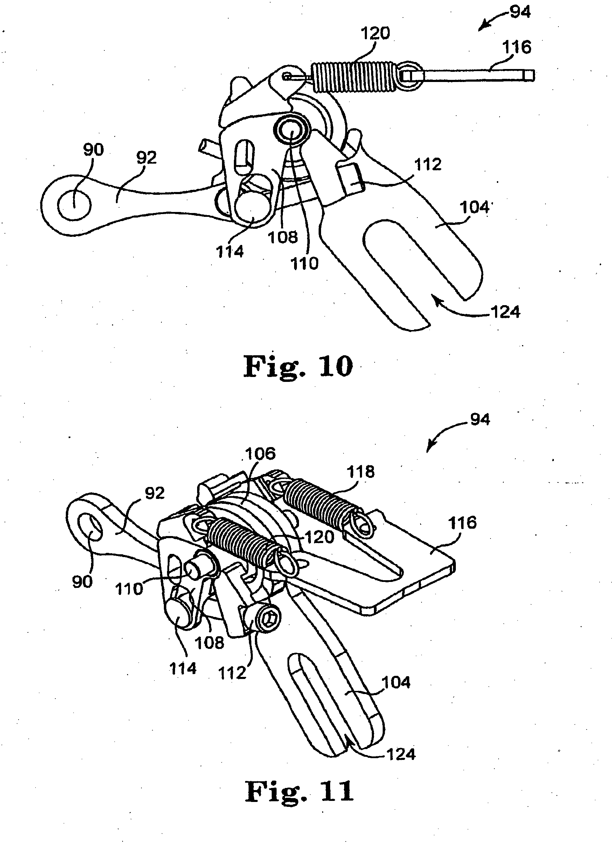

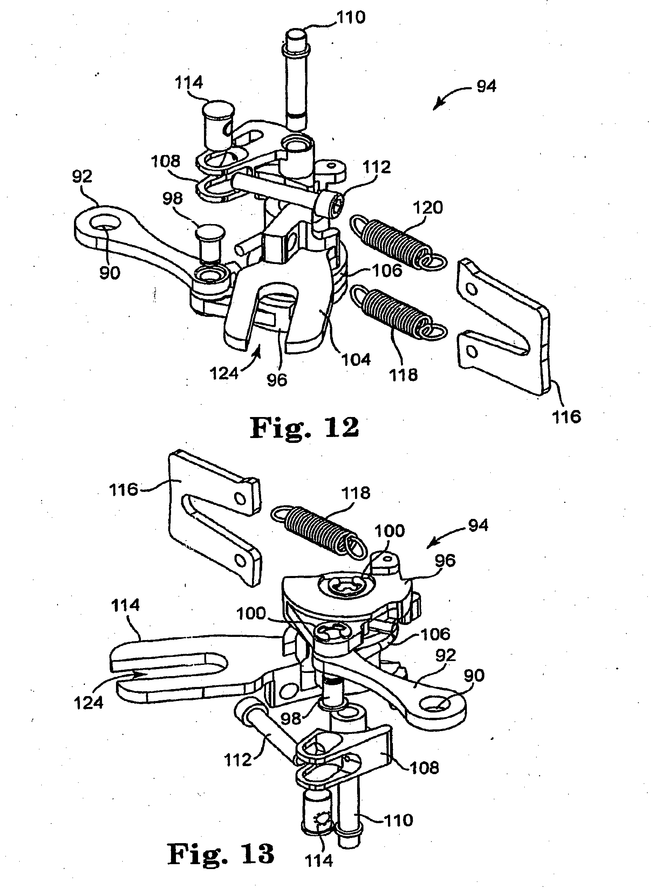

In particular, Figs 10-13 show that the clutch assembly 94 includes the link arm 92 which is attached distally to the wire terminal 82 (as discussed above) and proximally to a clutch 96 using a pin 98 and a clip 100 (Fig. 13) with the pin 98 (Figs. 12, 13) extending through an appropriately sized and shaped aperture 102 on the link arm 92 and an aperture (not shown) on the clutch 96, which are both coaxially aligned. The purpose of the clutch 96 is to move the link arm 92, which in turn moves the pull wire 35. The clutch 96 is preferably also attached to a clutch (or torsion) spring 106 (shown in Figs. 8-13). Preferably, a rotor 108 is attached to the clutch spring 106 opposite the clutch 96, with the rotor 108 including a screw 112 and anchor 114 to adjust the tension in the clutch spring 106. The cam 104 is attached to the rotor 108. As shown in the figures, the cam 104 includes a slot 124 into which the jaw activation lever 122 is moveably retained. There is an axle 110 running through apertures in the clutch 96, the cam 104 and the rotor 108, with the axle 110 being held in place using another clip 100.

The clutch spring 106 tension may be adjusted by tightening or loosening the screw 112 and anchor 114. In particular, in the embodiment shown in the figures, tightening the screw 112 will wind the clutch spring 106 tighter.

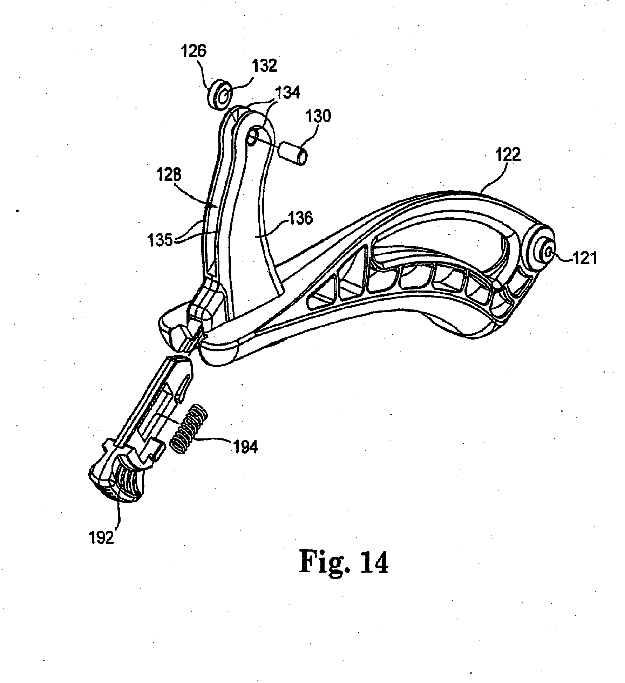

Referring to Figs. 8, 9, and 14, the handle assembly 24 also comprises the jaw activation (or closure) lever 122, which includes an extension portion 136 that is moveably attached to the cam 104 of the clutch assembly 94. The exemplary attachment of the extension 136 of the lever 122 shown is made by fitting a slot 124 of the cam 104 around a roller 126 in the extension 136 (Fig. 14), which is placed in a groove 128 in the extension 136 of the lever 122 and held in place using a pin 130 placed through an aperture 132 and two apertures 134 in the extension 136, which are coaxially aligned. The roller 126 of the jaw activation lever 122 is then able to roll along the slot 124 in the cam 104, allowing the two to move with respect to one another, in a predetermined path, while staying moveably connected. The lever 122 pivots about a point 121, where the lever 122 attaches to the handle casing 86. The lever 122 is preferably ergonomically shaped to fit in the hand of a user.

In order to activate, or close the jaws 28a, 28b, the lever 122 is squeezed or otherwise moved toward the handle casing 86. Moving the lever 122 in such a way results in the extension portion 136 of the lever 122 moving into the handle casing 86, which in turn pivots the cam 104 counter-clockwise (as in Figs. 8, 9) which through the components of the clutch assembly 94 pivots the clutch 96 counter clockwise (as in Figs. 8, 9). As a result, the clutch 96 pulls the link arm 92 generally proximally, and the wire terminal 82 moves proximally as well along the path of the recesses (one is 89a) in the handle casings 86a, 86b. Accordingly, the pull wire 35, attached to the wire terminal 82, is pulled proximally into the handle assembly 24, thereby closing the jaws 28a, 28b. Fig. 15 illustrates the positions of the handle 24 components when the jaws 28a, 28b are in a substantially closed position.

With the jaws 28a, 28b in a closed position, the components of the handle assembly 24 generally resemble Fig. 15. If further force is placed on the lever 122 (i.e., lever 122 is lifted or squeezed farther toward the handle casing 86), the overdrive slip described above prevents further tension from being placed on the pull wire 35. However, preferably, the lever 122 is moved toward the handle casing 86 further in order to lock the lever 122 in place, which in turn locks the jaws 28a, 28b in a locked position. Once the jaws 28a, 28b are locked in the closed position, a lockout feature of the present invention is deactivated, allowing for ablative energy to be applied. Such a lockout feature will be discussed in detail below.

The jaw closure mechanism described above is one exemplary such mechanism. It is also contemplated by the present invention that the jaws 28a, 28b may be driven by either a mechanical mechanism, e.g., a drive cable or wire in a compression jacket, a hydraulic mechanism, e.g., a piston powered by fluid pressure, and/or an electrical mechanism, e.g., a servo motor. Each of the jaw closure mechanisms described above would allow neck 22 to remain flexible or floppy when the jaws 28a, 28b were either in an open position and/or a closed position.

In the present invention, preferably the ablation device 12 includes a mechanism for preventing inadvertent application of ablative energy, which is referred to as a lockout mechanism or feature. In order to avoid inadvertent ablation, the lockout mechanism is preferably incorporated into the handle assembly 24. An example of such a lockout mechanism is included in the embodiments shown in Figs. 8, 9, and 15, and functions by preventing an ablative energy source from being activated unless the jaws 28a, 28b are locked in a substantially closed position.

Before the jaws 28a, 28b are locked in a closed position, some components of the handle assembly 24, in the exemplary device 12, prevent ablative energy from being applied. In particular, the exemplary embodiment prevents ablative energy from being applied by preventing a trigger 140 on the device 12 from being pulled. The mechanism for preventing the trigger 140 from being pulled to apply ablative energy may be referred to as a lockout mechanism. In the lockout mechanism illustrated, there is preferably a visual and/or tactile lockout flag 142 on or near the trigger 140 that indicates when the lockout mechanism is engaged or activated. While the lockout mechanism is activated, the lockout flag 142 extends through an aperture in the trigger 140 and can be seen and felt on the trigger 140, and when deactivated the lockout flag is recessed in the aperture in the trigger 140.

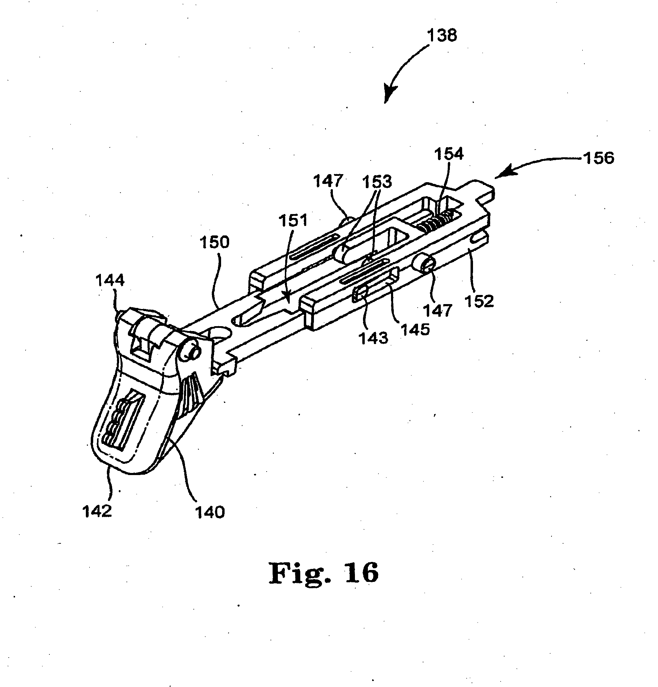

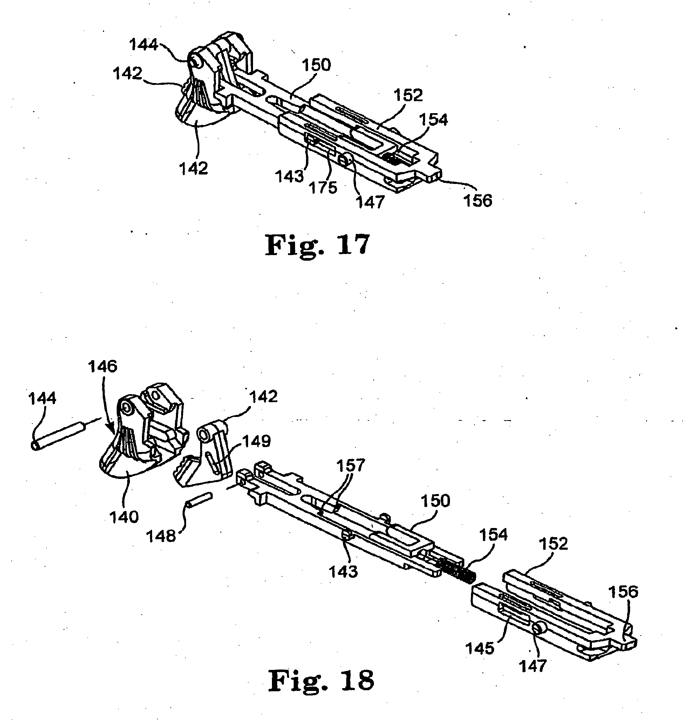

Additional components of the exemplary lockout mechanism can be seen separately in Figs. 16-18. These components are parts of a power trigger subassembly 138 which comprises the trigger 140 that is pivotally attached to the lockout flag 142 by a pin 144. The lockout flag 142 is attached via a slot 149 (Fig. 18) and a pin 148 that connects to a lockout slider 150. The lockout slider 150 is slidably retained in a lockout rail 152 with notches 143 on the sides of the slider 150 and channels 145 on the sides of the rail 152 in which the notches 143 may slide and a spring 154 between the rail 152 and slider 150, holding them apart on the proximal end of the power trigger subassembly 138. Also, on the proximal end of the rail 152, there is an extension or tail 156, which may depress a power switch to turn on the ablative energy source.

The power trigger subassembly is incorporated into the remainder of the handle assembly 24 as seen in Figs. 8, 9, and 15. The pin 144 that allows the trigger 140 to pivot with respect to the lockout flag 142 is also connected to the two handle casing halves (one half of which is shown as 86a). Also, bosses 147 on the outer sides of the rail 152 are connected to the handle casing halves (one is 86a) such that the rail may rotate or tilt with respect to the handle casing (one half of which is 86a). The components, therefore, generally allow the slider 160 to move proximally and distally within the rail that is attached to the handle casing. The slider 150 may pull the lockout flag 142 proximally which retracts the flag 142 into the trigger 140 and allows the trigger 140 to be free to rotate about pin 144. When the trigger 140 is free to rotate about pin 144, it may then be pulled or depressed such that the trigger 140 pushes up on the slider 150, which in turn causes the rail 152 to pivot or rotate about the bosses 147 such that the extension or tail 156 on the rail 152 may press on the power switch 164 to activate ablative energy application. By releasing the trigger 140, a torsion spring 162 pushes down on the rail 152 which pivots or rotates about the bosses 147. This causes the slider 150 to push down on the trigger 140, which will rotate about pin 144, which in turn causes the extension or tail 156 on the rail 152 to release pressure on the power switch 164 with further deactivates ablative energy application.

In order to move the slider 150 proximally to cause deactivation of the lockout mechanism, referring to Figs. 8 and 15, the extension 136 of the jaw activation lever 122 moves through slot 151 in the slider 150 (as lever 122 is squeezed) until proximal surfaces 137 of the extension 136 contact the proximal surfaces 153 in slot 151 in the slider 150, which moves the slider 150 proximally with respect to the rail 152 and in turn pulls the lockout flag 142 proximally so that the lockout flag 142 is recessed in the trigger 140.

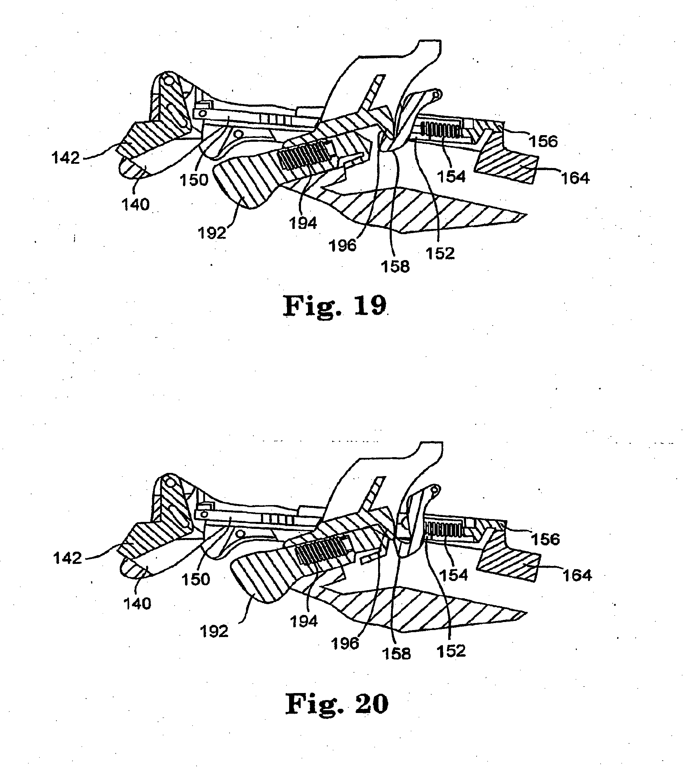

When the lockout flag 142 is recessed enough in order for the trigger 140 to be depressed, the lever 122 is also locked into the handle casing (one half of which is 86a). In the exemplary embodiment shown, a pawl 158 is attached to the handle casing (one half of which is 86a) and extends through slot 151 in the slider 150. The pawl 158 also has a tension spring 160 attached proximally. The lever 122 may be locked in the squeezed position when as the extension 136 is moving into the handle casing (one half of which is 86a) the pawl 158 catches on a projection 196 in the extension 136, which can be seen in the cross section of Fig. 19. With the pawl 158 caught on the projection 196, and with the lockout mechanism deactivated as described above, the trigger 140 may be depressed, which causes the rail 152 to pivot such that the extension 156 on the rail 152 depresses the power switch 164. The power switch 164 is preferably connected to a power source that is preferably located remotely from the handle assembly 24.

In order to release the jaw activation lever 122, open the jaws 28a, 28b on the jaw assembly 20, and reactivate the lockout mechanism, a lever release button 192 (Fig. 14) disposed in the lever 122 is pressed, squeezed for otherwise moved proximally into the lever 122. A cross-section of a portion of the handle portion 24 is shown in Fig. 20 showing what happens after the lever release button 192 has pushed the pawl 158 proximally so it is not caught on the projection 196 on the lever 122. As a result, the pawl 158 is pushed proximally and releases the jaw activation lever 122. The jaw activation lever 122 then moves away from the handle casing (one half of which is 86a) and the jaws 28a, 28b, are allowed to open. Distal surfaces 135 of extension 136 maintain contact with distal surfaces 157 of slider 150 and drive the slider 150 distally, which pushes the lockout flag 142 and causes it to pivot about the axis of pin 144. As a result, the lockout flag 142 extends through the aperture 146 in the trigger 140. In the preferred embodiment shown, the presence of the lockout flag 146 in such a position indicates both visually, as well as tactilely, to a user that the lockout mechanism is engaged and that ablative energy may not be applied.

The lockout mechanism illustrated in the figures and described above is one example of such a mechanism that prevents ablating energy from being inadvertently applied at an undesired location in a body. Other lockout mechanisms that prevent such inadvertent or accidental application of ablating energy at an undesired location in a body are also contemplated by the present invention. For example, it is contemplated that a lockout mechanism may be controlled through feedback from any number of sensors on the device, and in particular on the jaws of the device. Such sensors, could for example, sense whether or not they are clamped on desired tissue, which could in turn deactivate the lockout mechanism and allow ablative energy to be turned off and on. Any suitable feedback mechanisms are contemplated by the present invention for use in a lockout mechanism.

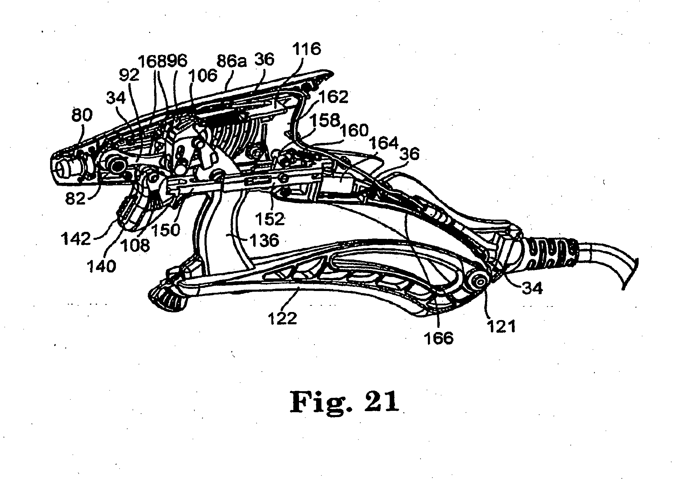

In order to supply power and fluid to the fluid assisted elongate electrode assembly that is preferably part of the ablation device 12, power source wires 34 and fluid delivery conduits 36 need to extend from a power source and a fluid source through the handle assembly 24, neck 22 and into the jaw assembly 20. There is discussion above of the preferred route for the power source wires 34 and fluid delivery conduits 36 through the neck 22 and jaw assembly 20. In the handle assembly 24, a preferred route of the power source wires 34 and fluid delivery conduits 36 is illustrated in Fig. 21. As shown in Fig. 21, the handle halves (86a only shown) may be provided with a series of laterally extending, perpendicular internal walls 168 that may include slots and/or recesses for routing power source wires 34 and fluid delivery conduits 36 and that extend through the handle casing 86. The power source wires 34 and fluid delivery conduits 36 are routed from the proximal end of the handle assembly 24 to the distal end, where they travel through apertures in the proximal neck retainer barb 80, where they may continue on to the neck 22 and jaw assembly 20.

The power source and fluid source are preferably located remotely from the ablation device 12. As seen in Fig. 1, a cord assembly 26 is attached to the handle assembly 24 through which to provide the power and fluid. In the cord assembly 26, a power source supply cord 172 retains the power source wires 34, and a fluid source supply cord 170 retains the fluid delivery conduits 36.

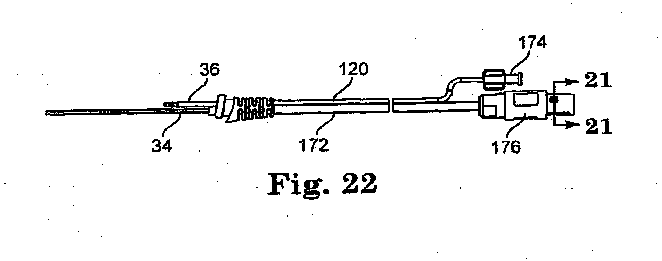

Fig. 22 shows the cord assembly 26 with a portion of the cords 170, 172 removed. It provides a closer view of connectors for the fluid and power sources. A fluid connector 174, such as that shown, preferably connects the fluid cord 170 to a fluid source. The female connector 174 is preferably a female luer, as shown. The fluid source may be a standard IV tubing system. In addition, the ablation device preferably includes a mechanism or device for controlling the amount of and the application of a fluid, such that the fluid is properly applied for fluid assisted ablation. A power connector 176 is also shown in Fig. 22, and preferably connects the power cord 172 to a power source. The cord assembly 26, and all components, shown and described are exemplary, and other suitable alternatives for delivering power and fluid to the handle assembly 24 are also contemplated by the present invention.

The ablation device 12 may incorporate one or more switches to facilitate regulation of one or more components or features of ablation device 12 by the operator. For example, one or more switches may control the supply of irrigation fluid and/or ablation energy to the jaw assembly 20 of ablation device 12. The one or more switches may be, for example, a hand switch, a foot switch and/or a voice-activated switch comprising voice-recognition technologies. The one or more switches may be incorporated on and/or in handle 24 of ablation device 12.

In the preferred embodiment shown in the figures, a power source switch 164 (e.g., RF switch) is included in the handle assembly 24 (Fig. 9). In order to supply power to the power source switch, and in order to activate or deactivate a remote power source from the power switch 164, there is preferably a set of wires 166 extending from the switch 164, out the proximal end of the handle assembly 24, and to a power source. Fig. 21 illustrates some exemplary wires 166 leading from the power switch 164, which may extend proximally through the power cord 172 to the power source.

Although not illustrated in the figures, the ablation device 12 may include one or more sensors or sensing elements to monitor one or more components or features. For example, preferably, the ablation device 12 may have the capability to monitor transmurality of ablation lesions. An example of a preferred algorithm used to monitor transmurality is disclosed in co-pending Provisional Patent Application, having Serial Number 60/832,242.

The ablation device 12 described above may, preferably, be part of a.system 10 (Fig. 1) for guiding the ablation device 12 to a desired location in a body. Other components of such a system 10 may comprise the first and second guide members 14, 16 and the guide member adapter 18. Detail about the guide members 14, 16 and the guide member adapter 18 is provided below with regard to the discussion of methods of using the ablation device 12 and methods of guiding the ablation device 12 into a location in a body.

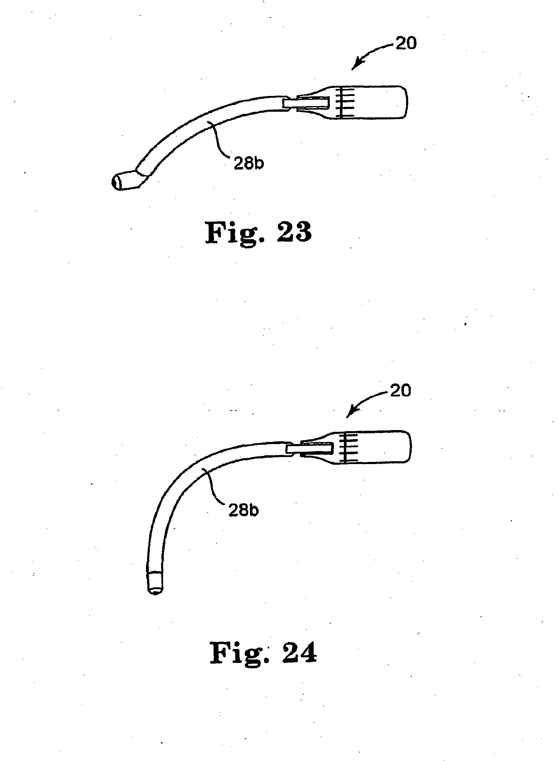

As part of a system 10 for guiding the ablation device 12 to a desired location in a body, the ablation device may include different jaw assemblies 20 for attachment to the neck 22 of the device 12. In particular, different jaw assemblies 20 that may be provided in such a system 10 may have jaws 28a, 28b with different curvatures or shapes. A purpose of having such different jaw assemblies is to accommodate different ablation procedures at different anatomical locations, as well as to accommodate the differing anatomy of individual patients. Figs. 23 and 24 show side views of two different embodiments of the jaw assembly 20 of the present invention. The two illustrative embodiments show clamping jaws 28a, 28b having different curvatures. As described above, a purpose of different curvatures may be to accommodate different ablation procedures. For example, the embodiment ofthe clamping jaws 28a, 28b (28b only shown) in Fig. 23 is preferably suited for a box lesions approach to pulmonary antrum isolation, as shown in Fig. 25 (on heart 174). Moreover, the embodiment of the clamping jaws 28a, 28b (28b only shown) shown in Fig. 24, which includes more curvature than those in Fig. 23, is preferably suited for a encircling island lesions approach to pulmonary antrum isolation, as shown in Fig. 26. The purpose of having clamping jaws 28a, 28b with more curvature for the encircling island lesions approach (Fig. 26) is to allow the clamping jaws 28a, 28b to be placed on tissue close to where the pulmonary veins end and the left atrium begins. Both the box lesions and encircling island lesions approaches will be described in more detail below with regard to the methods described below. In Figs. 25 and 26, although two sets of clamping jaws 28a, 28b are shown clamped or closed around or near both sets of pulmonary veins, this would preferably not be done simultaneously while a heart is off-pump. Generally, ablation of only one set of pulmonary veins is performed at a time so that blood flow is not occluded in the other set of pulmonary veins.

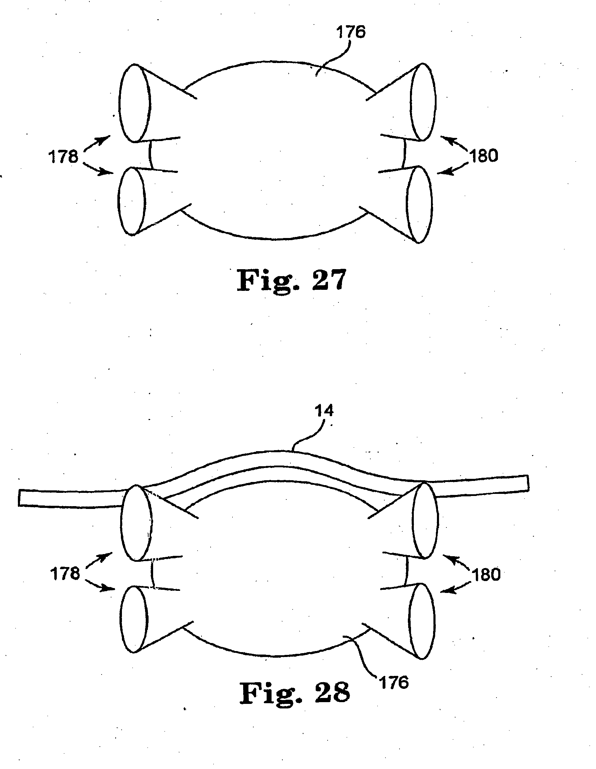

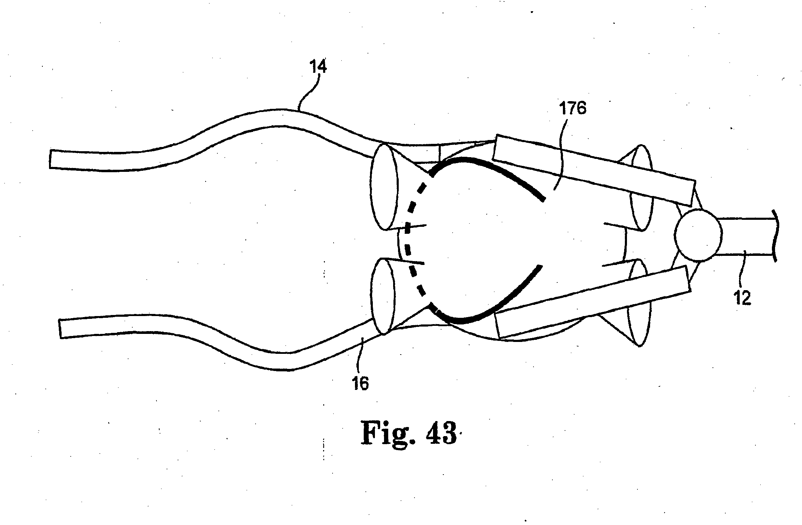

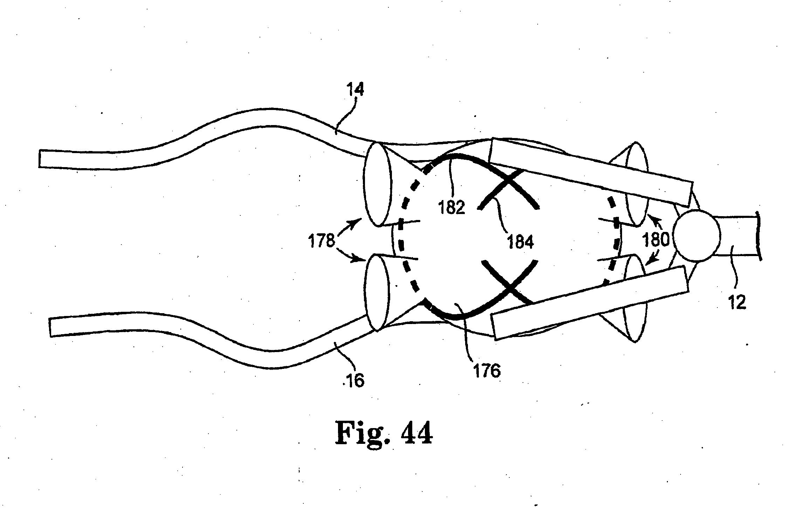

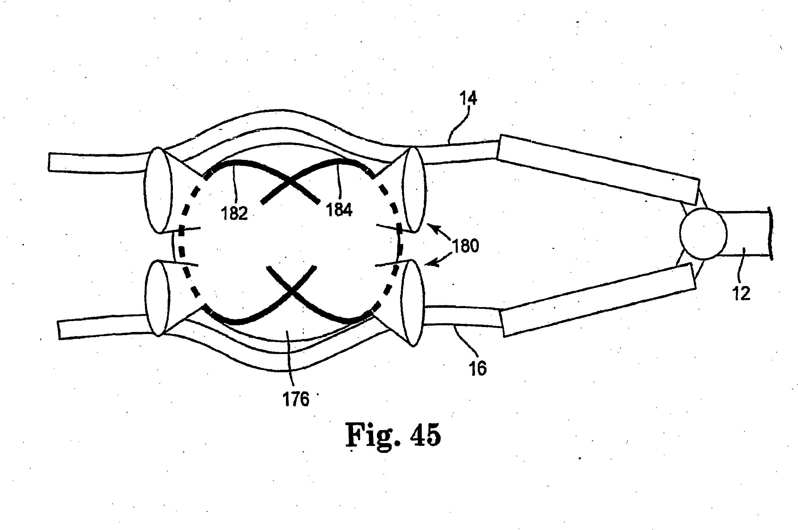

The ablation device 12 and/or system 10 may be used in ablation procedures in various areas in a body where ablation of tissue is desired. In particular, the ablation device 12 and system 10 is suitable for use in pulmonary antrum isolation. As described above there are different surgical approaches to pulmonary antrum isolation. With reference to Figs. 27-64, detail regarding use of the ablation device 12 and/or system 10, in accordance with the present invention, in both box lesions and encircling island lesions approaches to pulmonary antrum isolation is provided below. Figs. 27-30, 35-37, 41-47, 50-64 are schematic illustrations, and may not be anatomically correct or drawn to scale. The figures are provided to help in understanding methods of the present invention.

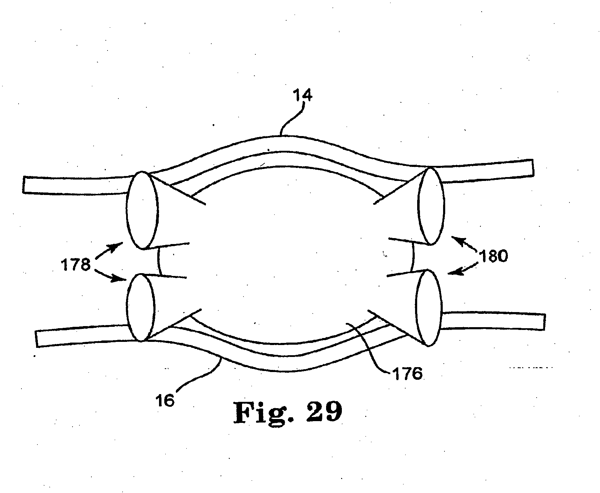

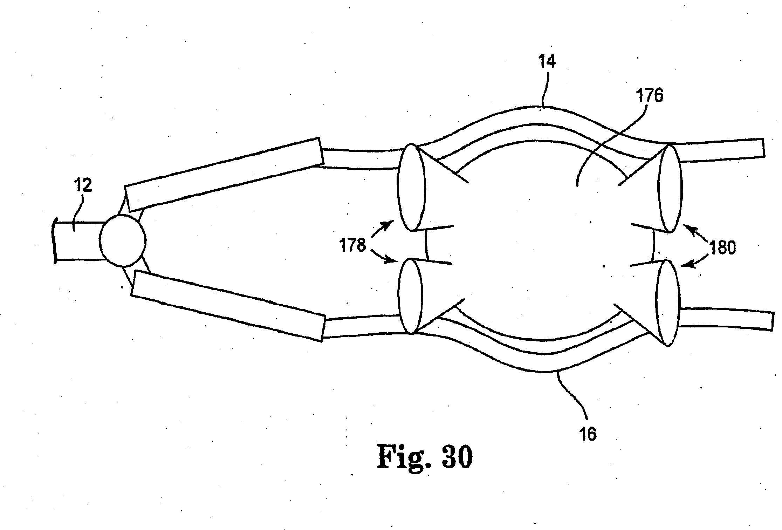

Figs. 27-46 illustrate steps in a method of using the ablation device 12 and/or guiding the ablation device 12, with the surgical approach being a box lesions approach. Fig. 27 illustrates schematically a pulmonary ostium 176 with the view being from the posterior side of a body and heart. The pulmonary ostium 176 includes two sets of pulmonary veins, right 180 and left 178. Fig. 28 shows a step in a method of guiding and using an ablation device 12, in accordance with the present invention, in which a first guide - member 14 is inserted posterior to the upper right and left pulmonary veins. The guide member 14 may be inserted through incisions in a minimally invasive procedure, for example. Four incisions or ports may be necessary in the procedure, allowing access to the pulmonary ostium 176 from four directions. The guide member, in this step and any other step described below, may be placed posterior to the upper right and left pulmonary veins using any known or future developed technique and/or device. Fig. 29 shows a subsequent step in the method in which a second guide member 16 is inserted posterior to the lower right and left pulmonary veins. Fig. 30 shows another subsequent step in which an ablation device 12 is attached at both distal ends of the jaws 28a, 28b to the first 14 and second 16 guide members.

The guide members 14, 16 or device may comprise a length of single or multi-lumen tubing, for example. An active guide connection may be included which has a connector member or device 216, e.g., a ball-in socket fitting, a luer fitting and/or a suture, located at one or more ends of the guide members 14, 16, for connection between the distal end portion of an ablation device (shroud 30). The guide members 14, 16 may include reference markings, to provide, for example, depth or length references. The guide member 14 or 16 may comprise two or more lengths of tubing, and the separate tubing sections may be color coded to facilitate differentiation between each other. In one embodiment, the guide member 14 or 16 may be used to safely pull the jaws 28a, 28b of the ablation device 12 into place if the neck 22 of the ablation device 12 is loose or floppy, e.g., the user cannot actively push or poke the jaws 28a, 28b into tissue, thereby causing undesirable tissue damage. The guide member 14 or 16 may include one or more blunt ends. The guide member 14 or 16 may include a suture on its distal end. Fig. 1 includes the guide members 14, 16 with sutures 15 on both ends. The suture(s) may be made of any suitable suture material. The purpose of the suture is to allow another instrument (e.g., forceps) to easily grab the suture on the end of the guide member 14 or 16 and pull the guide member 14 or 16 into a desired location. Also, the guide member 14 or 16 may include a wire backbone and an active guide connector.

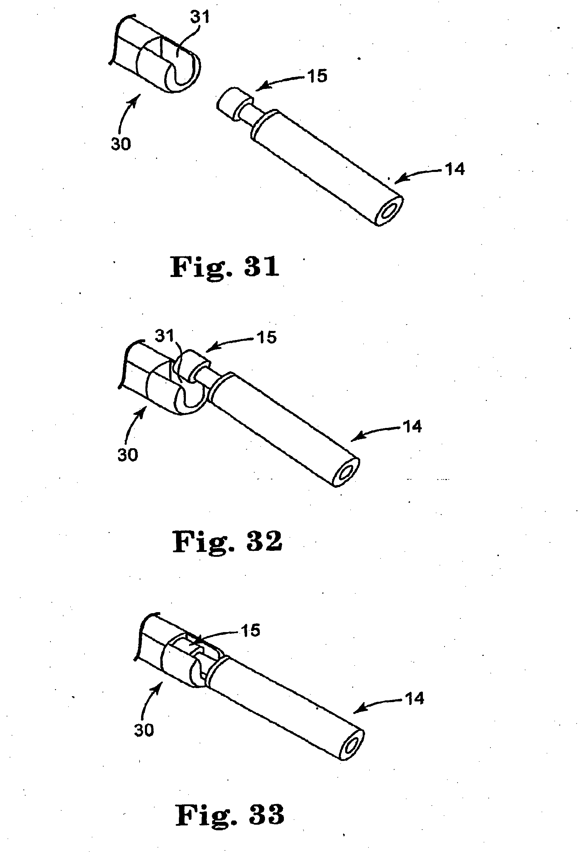

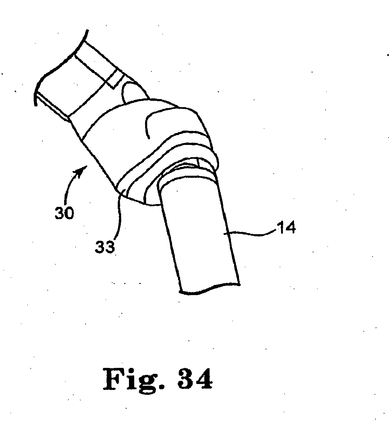

In order to attach the first and second guide members 14, 16 to the distal ends of the jaws 28a, 28b of the ablation device 12, an attachment means, such as that illustrated in Figs. 31-33 may be used. Fig. 31 shows the distal end of a jaw, which is called a shroud 30. The end of the guide member 14 includes a barb 15 that is shown in Fig. 32 as being fit into a socket 31 in the shroud. Preferably, the barb will fit into the socket 31 when the guide member 14 or 16 is held at an angle and maneuvered into the socket. Fig. 33 shows the final connected configuration. Fig. 34 shows another embodiment of the shroud 30 in which a covering 33 to the shroud 30 is provided. A purpose of the shroud 30 may be to prevent the barb 15 and other parts of the shroud 30 from catching on tissue as the ablation device 12 and guide member 14 are pulled through a body.

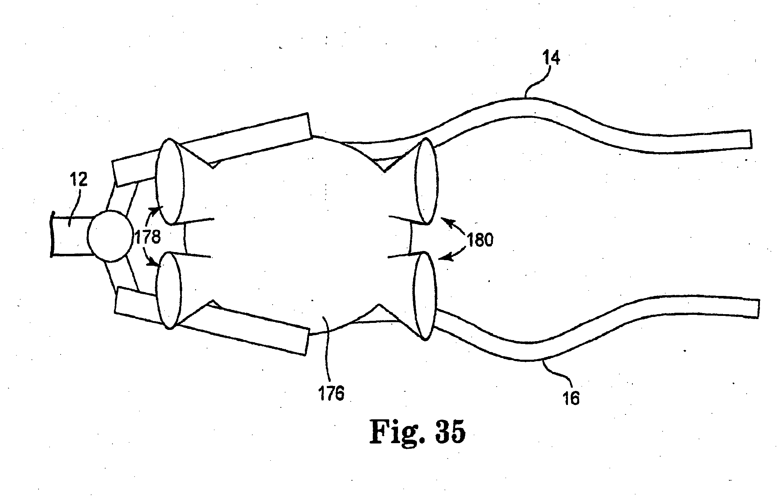

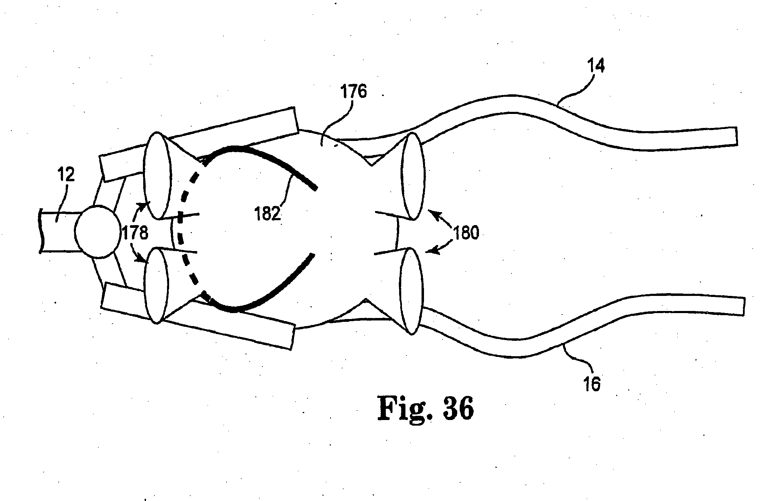

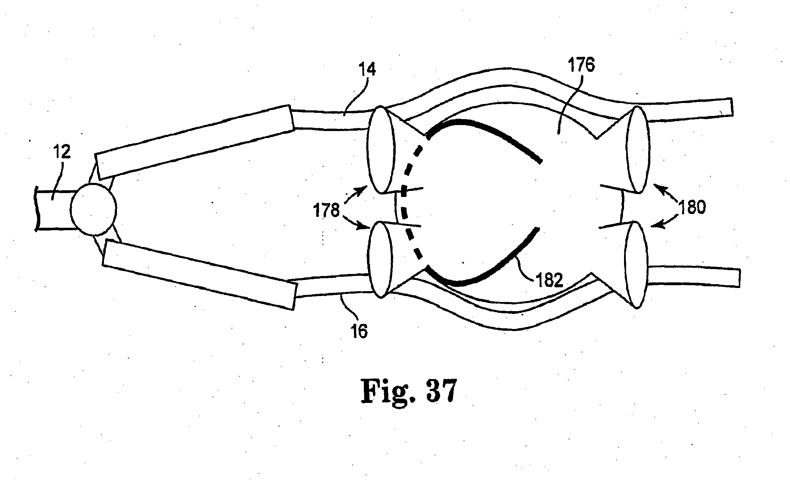

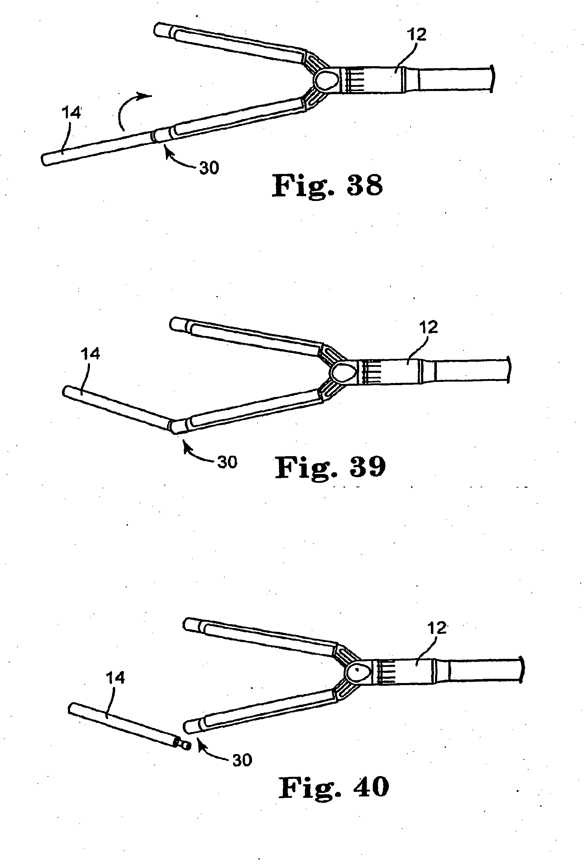

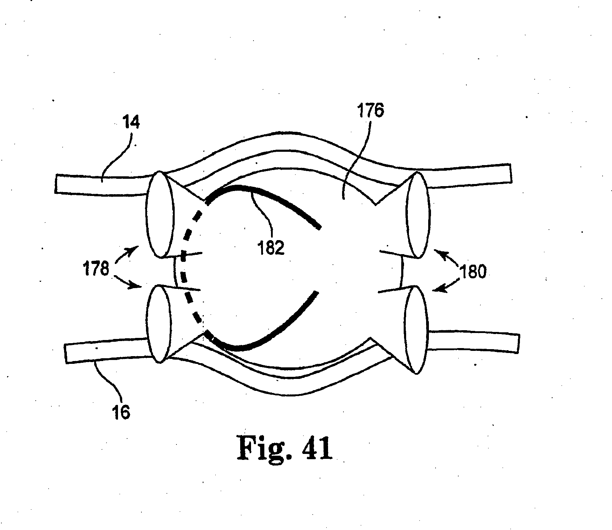

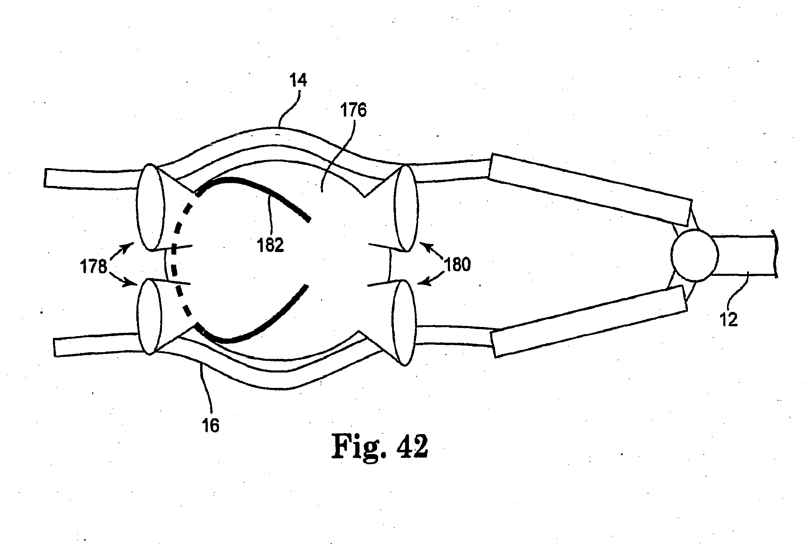

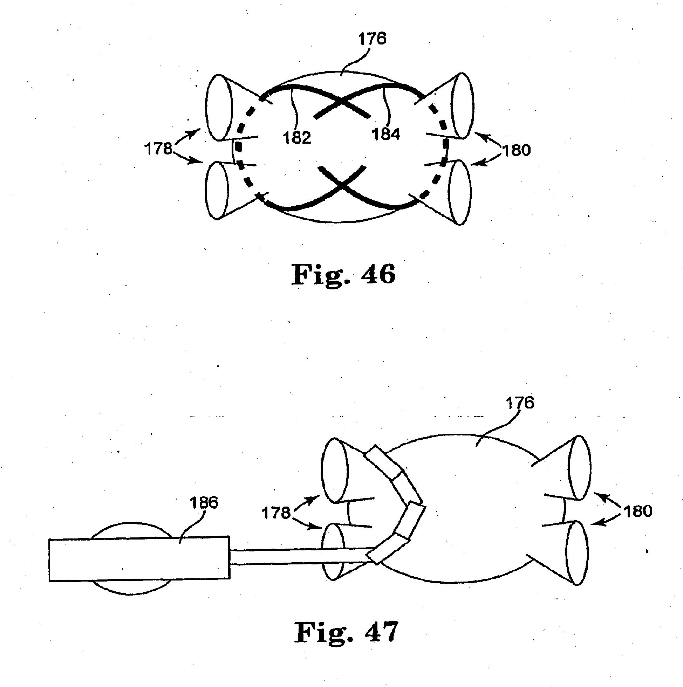

Fig. 35 illustrates a subsequent step in the method, in which the ablation device 12 is pulled into place with the jaws 20 surrounding the left set of pulmonary veins 178. The ablation device 12 is pulled into place by pulling the ends of the guide members 14, 16, which are opposite the ends to which the ablation device 12 is attached, out through the incisions or ports on the right side of the body. The next step, not illustrated, is to clamp the jaws 28a, 28b of the ablation device 12 on the surrounded tissue and activate the ablative energy to cause ablation. Fig. 36 illustrates a subsequent step in which the jaws 28a, 28b are opened and a lesion 182 can be seen that encircles the left pair of pulmonary veins 178. The next step, as in Fig. 37, is to withdraw the ablation device 12 back out of the body. The guide members 14 and 16 still attached. In order to remove the guide members 14, 16, the method depicted in Figs. 38-40 may be used. As can be seen in Figs. 38-40, the guide member 14, may be moved toward the interior of the jaws 28a, 28b, as indicated by arrow in Fig. 38. The guide member 14 may be removed from the shroud 30 after the guide member 14 is moved or tilted inward a certain degree. Fig. 41 illustrates the subsequent step after removal of the ablation device 12, in which the guide members 14, 16 are returned to their starting position, and the lesion 182 remains. The steps for ablating the left two pulmonary veins are then repeated on the left side of pulmonary ostium 176 (in Figs. 42-45), and the result is the pulmonary ostium, as seen in Fig. 46, with two overlapping lesions 182, 184.

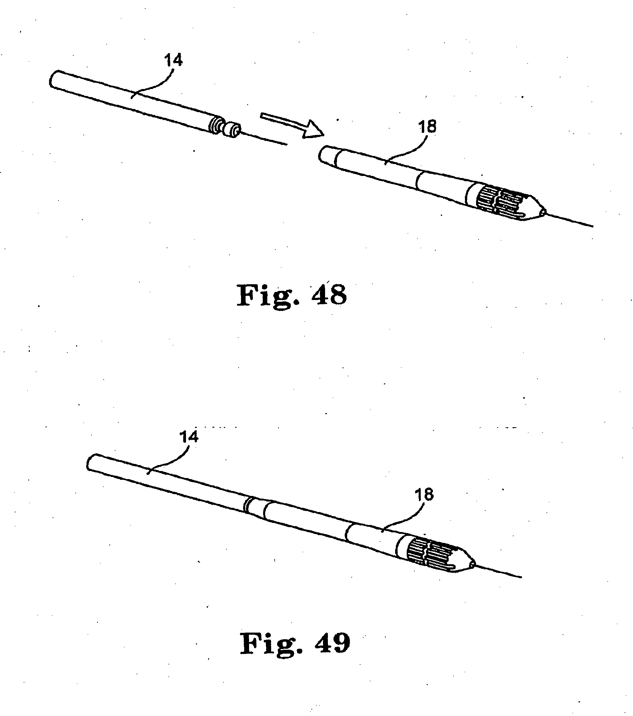

Fig. 49 illustrate steps in a method of using the ablation device 12 and guiding the ablation device 12 with a dissector/guide 186, with the surgical approach being an encircling island lesions approach. In a first step of the method, the dissector/guide 186 is inserted into a port of incision for minimally invasive procedures (but may also be used for open procedures).

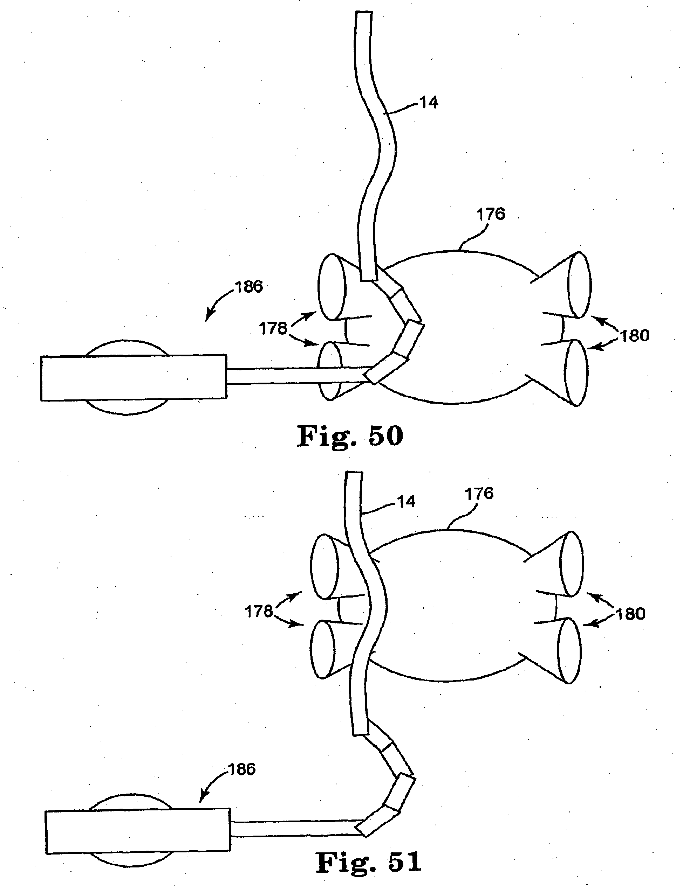

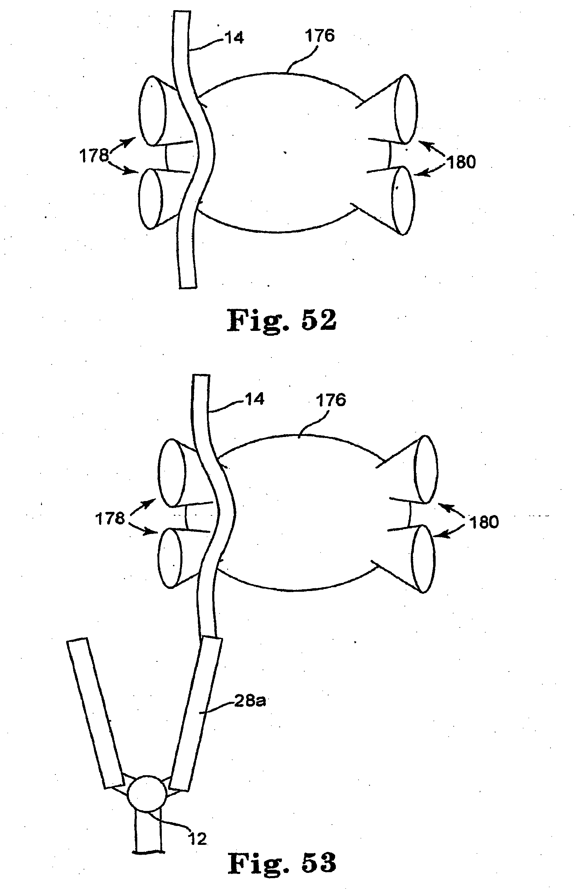

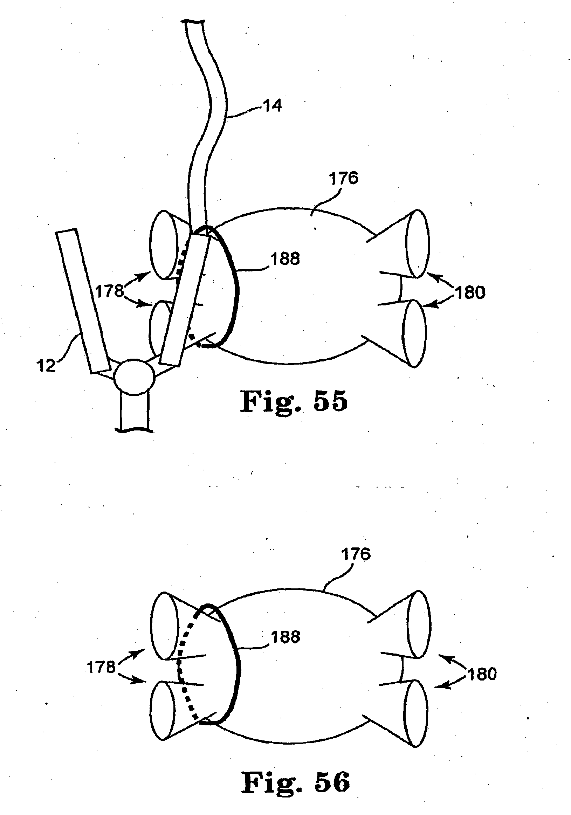

Fig. 47 shows the dissector/guide 186 articulated around the left pair of pulmonary veins 178. As described in the co-pending application described immediately above, a guide wire may be fed through the dissector/guide 186, and extended out another incision or port from one used for entry of the dissector/guide 186. The guide wire may then be attached to the guide member 14, and withdrawn back through the dissector/guide 186, which may pull the guide member 14 into contact with the distal end of the dissector/guide 186, as shown in Fig. 50. In the system 10 of the present invention, the guide member may have a guide member adapter 18 attached to an end of the guide member 14 in order to allow the guide wire to be attached. In Figs. 48-49, a guide wire adapter is shown, and with a guide member 14 inserted into the guide member adapter 18, which allows the guide wire to be attached to guide member 14 (through adapter 18). The next step is shown in Fig. 51, in which the dissector/guide 186 is withdrawn through its port of entry and pulls the attached guide member into the desired location surrounding the pulmonary veins as shown. In a subsequent step, the dissector/guide 186 is removed from the guide member 14, leaving the guide member 14 in place (Fig. 52). A subsequent step, shown in Fig. 53 illustrates an ablation device 12 being attached by one of its clamping jaws 28a to the guide member 14. The ablation device 12, next, is pulled into place around the pair of pulmonary veins 178 by pulling on the guide member 14. The jaws 28a, 28b are closed and ablative energy is applied to form a lesion 188, which can be seen in Fig. 55 after the jaws 28a, 28b were opened. After removal of the guide member 14 and ablation device 12, the lesion remains on the left side of the pulmonary ostium 176 (Fig. 56).

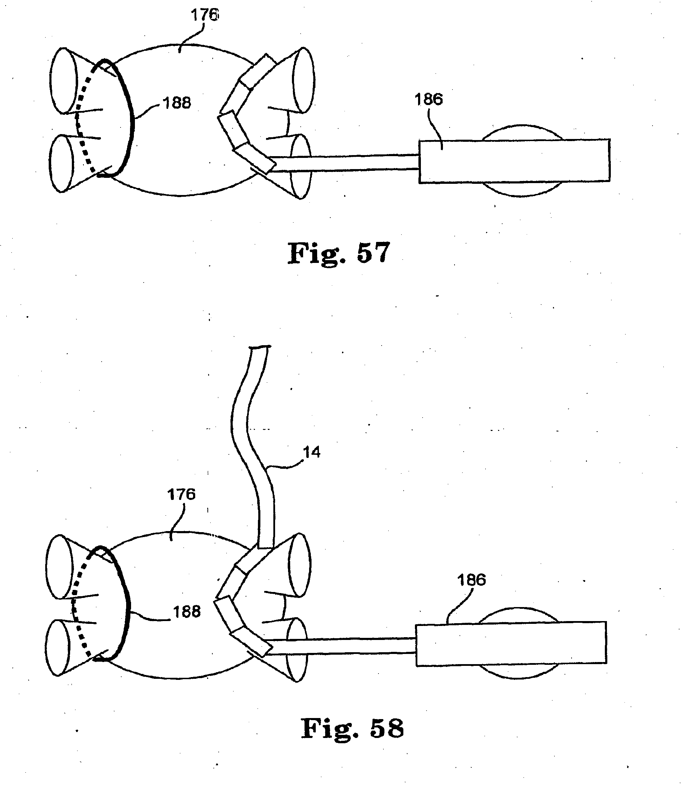

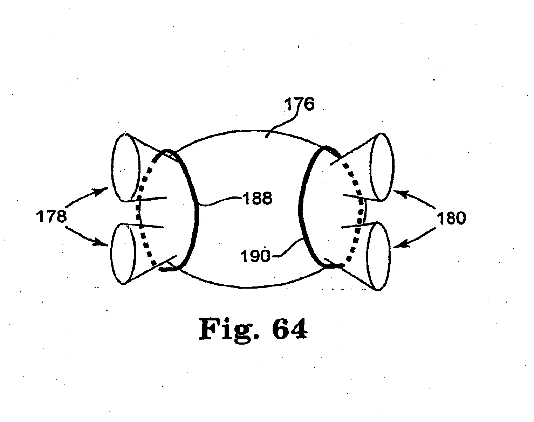

The steps for ablating the right two pulmonary veins are then repeated on the right side of pulmonary ostium 176 (in Figs. 57-63), and the result is the pulmonary ostium, as seen in Fig. 64, with two lesions 188, 190.

The ablation system 10 and its components are preferably made of biocompatible materials such as stainless steel, biocompatible epoxy or biocompatible plastic. Preferably, a biocompatible material prompts little allorgenic response from the patient's body and is resistant to corrosion from being placed within the patient's body. Furthermore the biocompatible material preferably does not cause any additional stress to the patient's body, for example, it does not scrape detrimentally against any element within the surgical cavity.

The invention is set out in the following claims.

## Claims

1. A device for ablating tissue at a desired location in a body, the device comprising:
a pair of floating jaws (28a, 28b), each having a proximal end and a distal end, moveable between a spaced apart open position and a closed position, the pair of jaws comprising at least one ablating element (52a, 52b) for ablating tissue located between the jaws;
a handle (24) comprising controls (34, 35, 36) for remotely controlling the movement of the jaws and the at least one ablative element;
a floppy neck (22) connecting the proximal ends of the jaws and handle, wherein the neck is flexible so as to permit the jaws to be maneuverable in the body with respect to the handle; and
first and second guide members (14, 16) removably attached to the distal ends of the jaws and configured to pull the jaws to a desired location in the body.

2. The device of claim 1, wherein the open position of the jaws is scissor-like in configuration and the closed position is parallel in configuration.

3. The device of claim 1, wherein the open position of the jaws is scissor-like in configuration and the closed position is parallel in configuration and as the jaws are moved from the open position to the closed position, the jaws follow a guided path including a first portion that causes the jaws to move in a scissor-like manner initially and then a second portion that causes the jaws to move in a parallel manner, wherein when following the guided path, the jaws are able to independently float to accommodate variations in tissue.

4. The device of claim 1, wherein the ablating element comprises a fluid assisted electrode assembly.

5. The device of claim 1 wherein the controls of the handle comprise a lever (122) that when moved causes the jaws to move between the open position and the closed position.

6. The device of claim 1, wherein the controls for the at least one ablating element comprise a trigger (140) that activates an ablative power source.

7. The device of claim 1, wherein the controls of the handle comprise a lock (104) for locking the jaws in the closed position.

8. The device of claim 1, wherein the controls of the handle comprise an overdrive mechanism for limiting closure of the jaws.

9. The device of claim 8, wherein the overdrive mechanism comprises a clutch assembly (94).

10. The device of claim 3, wherein the guided path comprises a slot on each jaw that is guided along a pin attached to the handle.

11. The device of claim 1, wherein the neck comprises an extruded polyurethane tube surrounding a stainless steel braid.

## Patentansprüche

1. Vorrichtung zur Ablation von Gewebe an einer gewünschten Stelle in einem Körper, wobei das Gerät Folgendes umfasst:
ein Paar gleitender Backen (28a, 28b), wobei jede davon ein proximales Ende und ein distales Ende aufweist, die zwischen einer voneinander beabstandeten, offenen Position und einer geschlossenen Position beweglich sind, wobei das Backenpaar wenigstens ein Ablationselement (52a, 52b) zur Ablation von Gewebe umfasst, das zwischen den Backen befindlich ist;
einen Griff (24), umfassend Kontrollgeräte (34, 35, 36) zur ferngesteuerten Regelung der Bewegung der Backen und des wenigstens einen Ablationselements;
einen schlaffen Hals (22), der die proximalen Enden der Backen und den Griff miteinander verbindet, wobei der Hals flexibel ist, um es den Backen zu erlauben, im Körper bezüglich des Griffs manövrierfähig zu sein; und
erste und zweite Führungselemente (14, 16), die entfernbar an den distalen Enden der Backen befestigt und derart konfiguriert sind, um die Backen an eine gewünschte Stelle im Körper zu ziehen.

2. Gerät nach Anspruch 1, wobei die offene Position der Backen eine scherenähnliche Konfiguration aufweist und die geschlossene Position eine parallele Konfiguration aufweist.

3. Gerät nach Anspruch 1, wobei die offene Position der Backen eine scherenähnliche Konfiguration aufweist und die geschlossene Position eine parallele Konfiguration aufweist und während die Backen von der offenen Position in die geschlossene Position bewegt werden, die Backen einem Führungsweg folgen, einschließlich einer ersten Position, welche die Backen dazu bringt, sich anfänglich in einer scherenähnlichen Weise zu bewegen und dann einem zweiten Abschnitt, der die Backen dazu bringt, sich auf parallele Weise zu bewegen, wobei beim Folgen des Führungswegs die Backen in der Lage sind, unabhängig voneinander zu gleiten, um den Variationen des Gewebes Rechnung zu tragen.

4. Gerät nach Anspruch 1, wobei das Ablationselement eine flüssigkeitsunterstützte Elektrodenanordnung umfasst.

5. Gerät nach Anspruch 1, wobei die Kontrollgeräte des Griffs einen Hebel (122) umfassen, der bei einer Bewegung die Backen dazu bringt, sich zwischen der offenen Position und der geschlossenen Position zu bewegen.

6. Gerät nach Anspruch 1, wobei die Kontrollgeräte für das wenigstens eine Ablationselement einen Auslöser (140) umfassen, der eine Ablations-Stromquelle aktiviert.

7. Gerät nach Anspruch 1, wobei die Kontrollgeräte des Griffs einen Verschluss (104) zum Verschließen der Backen in der geschlossenen Position umfassen.

8. Gerät nach Anspruch 1, wobei die Kontrollgeräte des Griffs einen Übersteuerungsmechanismus zum Limitieren des Verschlusses der Backen umfassen.

9. Gerät nach Anspruch 8, wobei der Übersteuerungsmechanismus eine Greiferanordnung (94) umfasst.

10. Gerät nach Anspruch 3, wobei der Führungsweg einen Schlitz an jeder Backe aufweist, der entlang eines am Griff befestigten Stifts geführt wird.

11. Gerät nach Anspruch 1, wobei der Hals einen extrudierten Polyurethanschlauch umfasst, der ein Geflecht aus Edelstahl umgibt.

## Revendications

1. Dispositif d'ablation de tissu en un emplacement voulu dans un corps, ce dispositif comprenant:
une paire de mâchoires flottantes (28a, 28b), chacune comportant une extrémité proximale et une extrémité distale, mobile entre une position ouverte espacée et une position fermée, la paire de mâchoires comprenant au moins un élément d'ablation (52a, 52b) permettant l'ablation de tissu situé entre les mâchoires ;
un manche (24) comprenant des commandes (34, 35, 36) permettant de commander à distance le mouvement des mâchoires et l'élément d'ablation au nombre d'au moins un ;
un cou souple (22) reliant les extrémités proximales des mâchoires et le manche, dans lequel le cou est flexible de manière à permettre de manoeuvrer les mâchoires dans le corps par rapport à la poignée ; et
des premier et deuxième éléments de guidage (14, 16) attachés de manière amovible aux extrémités distales des mâchoires et configurés pour tirer les mâchoires jusqu'à un emplacement voulu dans le corps.

2. Dispositif selon la revendication 1, dans lequel la position ouverte des mâchoires est en configuration de type ciseaux et où la position fermée est parallèle en termes de configuration.

3. Dispositif selon la revendication 1, dans lequel la position ouverte des mâchoires est en configuration de type ciseaux et où la position fermée est parallèle en termes de configuration et alors que les mâchoires sont déplacées de la position ouverte à la position fermée, les mâchoires suivent une voie guidée comprenant une première position qui amène les mâchoires à se déplacer en ciseaux au départ puis une deuxième position qui amène les mâchoires à se déplacer de manière parallèle, dans lequel lorsqu'on suit la voie guidée, les mâchoires sont à même de flotter indépendamment pour s'adapter aux variations rencontrées dans le tissu.

4. Dispositif selon la revendication 1, dans lequel l'élément d'ablation comprend un ensemble d'électrodes assisté par un fluide.

5. Dispositif selon la revendication 1, dans lequel les commandes du manche comprennent un levier (122) qui lorsqu'on le déplace, amène les mâchoires à se déplacer entre la position ouverte et la position fermée.

6. Dispositif selon la revendication 1, dans lequel les commandes destinées à l'élément d'ablation au nombre d'au moins un comprennent une détente (140) qui active une alimentation électrique d'ablation.

7. Dispositif selon la revendication 1, dans lequel les commandes du manche comprennent un verrou (104) permettant de verrouiller les mâchoires en position fermée.

8. Dispositif selon la revendication 1, dans lequel les commandes du manche comprennent un mécanisme de surmultiplication permettant de limiter la fermeture des mâchoires.

9. Dispositif selon la revendication 8, dans lequel le mécanisme de surmultiplication comprend un ensemble d'embrayage (94).

10. Dispositif selon la revendication 3, dans lequel la voie guidée comprend une fente sur chaque mâchoire, qui est guidée le long d'une broche attachée au manche.

11. Dispositif selon la revendication 1, dans lequel le cou comprend un tube extrudé de polyuréthane entourant une tresse d'acier inoxydable.
